# EUROPÄISCHE PATENTSCHRIFT

(11) **EP 0 465 621 B1**
(45) Veröffentlichungstag und Bekanntmachung des Hinweises auf die Patenterteilung: **13.11.1996**
(21) Anmeldenummer: 91902678.1
(22) Anmeldetag: 21.01.1991
(51) Int. Cl.: C07C 22/08, C07C 25/18, C07C 49/813, C09K 19/30, G02F 1/13

(54) **PARTIELL FLUORIERTE VERBINDUNGEN**
PARTIALLY FLUORINATED COMPOUNDS
COMPOSES PARTIELLEMENT FLUORES

(30) Priorität: 27.01.1990 DE 4002375; 27.01.1990 DE 4002376; 19.09.1990 DE 4029602
(43) Veröffentlichungstag der Anmeldung: 15.01.1992
(73) Patentinhaber: MERCK PATENT GmbH, D-64271 Darmstadt (DE)
(72) Erfinder: BARTMANN, Ekkehard, D-6106 Erzhausen (DE); POETSCH, Eike, D-6109 Mühltal (DE); FINKENZELLER, Ulrich, D-6831 Plankstadt (DE); RIEGER, Bernhard, D-6115 Münster (DE); PAULUTH, Detlef, D-6100 Darmstadt (DE)
(86) Internationale Anmeldenummer: EP9100102
(87) Internationale Veröffentlichungsnummer: WO9111418

(56) Entgegenhaltungen:
- EP-A- 414 048
- EP-A- 0 325 796
- EP-A- 0 351 587
- Patent Abstracts of Japan, unexamined applications, C Sektion, Band 11, Nr. 251, 14 August 1987 The Patent Office Japanese Government see pages 52 C 440 Kokai No. 62-56 444

## Beschreibung

Die Erfindung betrifft partiell fluorierte Verbindungen der Formel I,

R¹-A¹-Z¹-(A²-Z²)ₘ-A³-(Y)ₙ-Q-R² I

wobei
- A¹, A² und A³: jeweils unabhängig voneinander einen
(a) trans-1,4-Cyclohexylenrest, worin auch eine oder mehrere nicht benachbarte CH₂-Gruppen durch -0- und/oder -S- ersetzt sein können,
(b) 1,4-Phenylenrest, worin auch eine oder zwei CH-Gruppen durch N ersetzt sein können,
(c) Rest aus der Gruppe 1,3-Cyclobutylen, 1,3-Bicyclo(1,1,1)pentylen, 1,4-Cyclohexenylen, 1,4-Bicyclo(2,2,2)octylen, Piperidin-1,4-diyl, Naphthalin-2,6-diyl, Decahydronaphthalin-2,6-diyl und 1,2,3,4-Tetrahydronaphthalin-2,6-diyl,
wobei die Reste (a) und (b) durch CN oder Halogen substituiert sein können,
- R¹ und R²: jeweils unabhängig voneinander einen unsubstituierten oder einen einfach durch CN, Halogen oder CF₃ substituierten Alkyl- oder Alkenylrest mit bis zu 15 C-Atomen, wobei in diesen Resten auch eine oder mehrere CH₂-Gruppen jeweils unabhängig voneinander durch -S-, -O-, -CO-, -CO-O-, -O-CO- oder -O-CO-O- so ersetzt sein können, daß S- und/oder O-Atome nicht direkt miteinander verknüpft sind, einer der Reste R¹ und R² auch H,
- Z¹ und Z²: jeweils unabhängig voneinander -CH₂CH₂-, -CC-, -CH₂O-, -OCH₂-, -CO-O-, -O-CO-, -CH=N-, -N=CH-, -CH₂S-, -SCH₂-, eine Einfachbindung oder eine Alkylengruppe mit 3 bis 6 C-Atomen, worin auch eine CH₂-Gruppe durch -O-, -CO-O-, -O-CO-, -CHHalogen- oder -CHCN- ersetzt sein kann, und
- Y: O, S, CO, CO-O oder O-CO,
- Q: (CHF)ₒ-CF₂-(CHF)_{P}, im Falle n = O auch -CH₂-CF₂-, oder -CF₂CF₂- ,
- m: 0, 1 oder 2,
- n: 0 oder 1, und
- o, p: 0 oder 1
bedeuten,
mit der Maßgabe, daß falls R² H bedeutet, die Summe o + p 1 oder 2 ist.

Die Erfindung betrifft weiterhin die Verwendung dieser Verbindungen als Komponenten flüssigkristalliner Medien sowie Flüssigkristall- und elektrooptische Anzeigeelemente, die die erfindungsgemäßen flüssigkristallinen Medien enthalten.

Die Verbindungen der Formel I können als Komponenten flüssigkristalliner Medien verwendet werden, insbesondere für Displays, die auf dem Prinzip der verdrillten Zelle einschließlich deren hochverdrillten Varianten, wie z.B. STN oder SBE, dem Guest-Host-Effekt, dem Effekt der Deformation aufgerichteter Phasen oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Medien geeignet sind und insbesondere eine vergleichsweise geringe Viskosität besitzen sowie eine mittlere positive dielektrische Anisotropie.

Es wurde nun gefunden, daß Verbindungen der Formel I als Komponenten flüssigkristalliner Phasen vorzüglich geeignet sind. Insbesondere verfügen sie über vergleichsweise niedere Viskositäten. Mit ihrer Hilfe lassen sich stabile flüssigkristalline Phasen mit breitem Mesophasenbereich vorteilhaften Werten für die optische und dielektrische Anisotropie erhalten, welche sich gleichzeitig durch sehr günstige Werte für.den spezifischen Widerstand auszeichnen. Hierdurch lassen sich insbesondere bei Medien für Aktive-Matrix-Displays oder Supertwistdisplays deutliche Vorteile erzielen.

Ähnliche Verbindungen mit flüssikristallinen Eigenschaften mit terminalen Gruppen, welche eine (CH₂)ₙ-(CF₂)ₙ, eine CHF oder eine CF₂CF₂-Gruppierung aufweisen, sind bereits bekannt.

Aus der WO 88/05803 sind flüssigkristalline Mischungen bekannt, welche als Dotierstoffe zur Herabsetzung des Brechungsindexes dieser Mischungen Verbindungen mit (CH₂)ₙ-(CF₂)ₘ-Strukturelemente enthalten. Diese Verbindungen sind meist nicht flüssigkristallin und weisen mindestens eine Methylengruppe zwischen den Ringgliedern und den Perfluoralkylengruppen auf.

Aus der DE-OS-37 14 043 sind Flüssigkristallanzeigeelemente bekannt, die zur Herabsetzung der Schaltzeit fluorhaltige Verbindungen mit mindestens einer Tetrafluorethylengruppe enthalten, bekannt.

Flüssigkristalline Verbindungen mit einer Alkylgruppe, welche eine CHF-Gruppe aufweisen, sind z. B. aus der DE-OS-33 32 692 bekannt.Daneben werden solche optisch aktive Verbindungen als chirale Dotierstoffe für ferroelektrische flüssigkristalline Phasen eingesetzt, z. B. EP-A-0 237 007.

Mit der Bereitstellung von Verbindungen der Formel I wird außerdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Die erfindungsgemäßen Verbindungen der Formel I mit einer chiralen CF₂-CHF*-Gruppe eignen sich insbesondere als Dotierstoffe für chirale getiltete smektische Phasen mit ferroelektrischen Eigenschaften.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie stabil.

Gegenstand der Erfindung sind somit die Verbindungen der Formel I. Insbesondere solche Verbindungen der Formel I, worin
- R¹ und R²: jeweils unabhängig voneinander einen Alkylrest mit 1 bis 15 C-Atomen, und
- Q: CF₂
bedeuten, insbesondere solche
partiell fluorierte Verbindungen,
worin

(Y)ₙ-Q- CO-CF₂-

bedeutet.

Eine bevorzugte Ausführungsform stellen die Verbindungen der Formel I' und I" dar,
wobei
- n: 0,
- Q: CF₂, und einer der Ringe A² und A³ 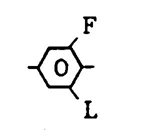
mit L = H oder F bedeuten.

Weiterhin sind insbesondere solche partiell fluorierte Verbindungen der Formel I, worin mindestens einer der Reste A¹, A² und A³ gegebenenfalls durch Fluor substituiertes 1,4-Phenylen, 1,4-Cyclohexylen, Pyrimidin-2,5-diyl oder Pyridin-2,5-diyl bedeutet, Gegenstand der Erfindung.

Eine weitere bevorzugte Ausführungsform dieser Erfindung sind partiell fluorierte Verbindungen der Formel I, worin R¹, A¹, A², Z¹, Z² und m die vorgegebene Bedeutung besitzen, n 0 ist, R² Alkoxy, Thioalkyl, Alkanoyl, Alkanoyloxy oder Alkoxycarbonyl mit 1 bis 14 C-Atomen bedeutet und A³ unsubstituiertes oder durch ein oder zwei Fluoratome substituiertes 1,4-Phenylen oder trans-1,4-Cyclohexylen bedeutet.

Gegenstand der Erfindung ist weiterhin die Verwendung dieser Verbindungen als Komponenten flüssigkristalliner Medien. Gegenstand der Erfindung sind ferner flüssigkristalline Medien mit einem Gehalt an mindestens einer Verbindung der Formel I sowie Flüssigkristallanzeigeelemente, insbesondere elektrooptische Anzeigeelemente, die derartige Medien enthalten.

Der Einfachheit halber bedeuten im folgenden X -(Y)ₙ-Q, -Cyc einen 1,4-Cyclohexylenrest, Che einen 1,4-Cyclohexenylenrest, Dio einen 1,3-Dioxan-2,5-diylrest, Dit einen 1,3-Dithian-2,5-diylrest, Phe einen 1,4-Phenylenrest, Pyd einen Pyridin-2,5-diylrest, Pyr einen Pyrimidin-2,5-diylrest und Bi einen Bicyclo(2,2,2)-octylenrest, wobei Cyc und/oder Phe unsubstituiert oder ein- oder zweifach durch F oder CN substituiert sein können.

Die Verbindungen der Formel I umfassen dementsprechend Verbindungen mit zwei Ringen der Teilformeln Ia bis Ib:

R¹-A¹-A³-X-R² Ia

R¹-A¹-Z¹-A³-X-R² Ib

Verbindungen mit drei Ringen der Teilformeln Ic bis If:

R¹-A¹-A²-A³-X-R² Ic

R¹-A¹-Z¹-A²-Z²-A³-X-R² Id

R¹-A¹-Z¹-A²-A³-X-R² Ie

R¹-A¹-A²-Z²-A³-X-R² If

sowie Verbindungen mit vier Ringen der Teilformeln Ig bis In:

R¹-A¹-A²-A²-A³-X-R² Ig

R¹-A¹-Z¹-A²-A²-A³-X-R² Ih

R¹-A¹-A²-Z²-A²-A³-X-R² Ii

R¹-A¹-A²-A²-Z²-A³-X-R² Ij

R¹-A¹-Z¹-A²-Z²-A²-A³-X-R² Ik

R¹-A¹-Z¹-A²-A²-Z²-A³-X-R² Il

R¹-A¹-A²-Z²-A²-Z²-A³-X-R² Im

R¹-A¹-Z¹-A²-Z²-A²-Z²-A³-X-R² In

Darunter sind besonders diejenigen der Teilformeln Ia, Ib, Ic, Id, Ie, If, Ig, Ih, Ii und Il bevorzugt.

Die bevorzugten Verbindungen der Teilformel Ia umfassen diejenigen der Teilformeln Iaa bis Iah:

R¹-Phe-Phe-X-R² Iaa

R¹-Phe-Cyc-X-R² Iab

R¹-Dio-Phe-X-R² Iac

R¹-Pyr-Phe-X-R² Iad

R¹-Pyd-Phe-X-R² Iae

R¹-Cyc-Phe-X-R² Iaf

R¹-Cyc-Cyc-X-R² Iag

R¹-Che-Phe-X-R² Iah

Darunter sind diejenigen der Formeln Iaa, Iab, Iac, Iad, Iaf und Iag besonders bevorzugt.

Die bevorzugten Verbindungen der Teilformel Ib umfassen diejenigen der Teilformeln Iba bis Ibo:

R¹-Phe-CH₂CH₂-Phe-X-R² Iba

R¹-Phe-OCH₂-Phe-X-R² Ibb

R¹-Cyc-CH₂CH₂-Phe-X-R² Ibc

R¹-Cyc-CH₂-CH₂-Cyc-X-R² Ibd

R¹-Cyc-COO-Phe-X-R² Ibe

R¹-Cyc-COO-Cyc-X-R² Ibf

R¹-A¹-CH₂CH₂-Phe-X-R² Ibg

R¹-A¹-CH₂CH₂-Cyc-X-R² Ibh

R¹-A¹-CH₂O-Phe-X-R² Ibi

R¹-A¹-OCH₂-Phe-X-R² Ibj

R¹-A¹-COO-Phe-X-R² Ibk

R¹-A¹-OCO-Phe-X-R² Ibl

R¹-Che-CH₂CH₂-Phe-X-R² Ibm

R¹-Phe-CC-Phe-X-R² Ibn

R¹-Phe-COO-Phe-X-R² Ibo

Darunter sind diejenigen der Formel Iba, Ibc, Ibe, Ibn und Ibo besonders bevorzugt .

Die bevorzugten Verbindungen der Teilformel Ic umfassen diejenigen der Teilformeln Ica bis Icm:

R¹-Phe-Phe-Phe-X-R² Ica

R¹-Phe-Phe-Cyc-X-R² Icb

R¹-Phe-Dio-Phe-X-R² Icc

R¹-Cyc-Cyc-Phe-X-R² Icd

R¹-Cyc-Cyc-Cyc-X-R² Ice

R¹-Pyd-Phe-Phe-X-R² Icf

R¹-Pyr-Phe-Phe-X-R² Icg

R¹-Phe-Pyr-Phe-X-R² Ich

R¹-Cyc-Phe-Phe-X-R² Ici

R¹-Cyc-Phe-Cyc-X-R² Icj

R¹-Dio-Phe-Phe-X-R² Ick

R¹-Che-Phe-Phe-X-R² Icl

R¹-Phe-Che-Phe-X-R² Icm

Darunter sind diejenigen der Formeln Ica, Icc, Icd, Ice, Ici und Icj besonders bevorzugt.

Die bevorzugten Verbindungen der Teilformel Id umfassen diejenigen der Teilformeln Ida bis Idm:

R¹-Phe-Z¹-Phe-Z²-Phe-X-R² Ida

R¹-Phe-Z¹-Phe-Z²-Cyc-X-R² Idb

R¹-Phe-Z¹-Dio-Z²-Phe-X-R² Idc

R¹-Cyc-Z¹-Cyc-Z²-Phe-X-R² Idd

R¹-Cyc-Z¹-Cyc-Z²-Cyc-X-R² Ide

R¹-Pyd-Z¹-Phe-Z²-Phe-X-R² Idf

R¹-Phe-Z¹-Pyd-Z²-Phe-X-R² Idg

R¹-Pyr-Z¹-Phe-Z²-Phe-X-R² Idh

R¹-Phe-Z¹-Pyr-Z²-Phe-X-R² Idi

R¹-Phe-Z¹-Cyc-Z²-Phe-X-R² Idj

R¹-Cyc-Z¹-Phe-Z²-Cyc-X-R² Idk

R¹-Dio-Z¹-Phe-Z²-Phe-X-R² Idl

R¹-Che-Z¹-Phe-Z²-Phe-X-R² Idm

Die bevorzugten Verbindungen der Teilformel Ie umfassen diejenigen der Teilformeln Iea bis Iek:

R¹-Pyr-Z¹-Phe-Phe-X-R² Iea

R¹-Dio-Z¹-Phe-Phe-X-R² Ieb

R¹-Cyc-Z¹-Phe-Phe-X-R² Iec

R¹-Cyc-Z¹-Phe-Cyc-X-R² Ied

R¹-Phe-Z¹-Cyc-Phe-X-R² Iee

R¹-Cyc-Z¹-Cyc-Phe-X-R² Ief

R¹-Cyc-Z¹-Cyc-Cyc-X-R² Ieg

R¹-Phe-Z¹-Dio-Phe-X-R² Ieh

R¹-Pyd-Z¹-Phe-Phe-X-R² Iei

R¹-Phe-Z¹-Pyr-Phe-X-R² Iej

R¹-Phe-Z¹-Che-Phe-X-R² Iek

R¹-Phe-Z¹-Phe-Phe-X-R² Iel

Darunter sind diejenigen der Formeln Iec, Ief und Iel besonders bevorzugt, insbesondere worin Z² -H₂CH₂-, -CO-O- oder -O-CO- bedeutet.

Die bevorzugten Verbindungen der Teilformel If umfassen diejenigen der Teilformeln Ifa bis Ifp

R¹-Pyr-Phe-Z²-Phe-X-R² Ifa

R¹-Pyr-Phe-OCH₂-Phe-X-R² Ifb

R¹-Phe-Phe-Z²-Phe-X-R² Ifc

R¹-Phe-Phe-Z²-Cyc-X-R² Ifd

R¹-Cyc-Cyc-Z²-Phe-X-R² Ife

R¹-Cyc-Cyc-Z²-Cyc-X-R² Iff

R¹-Cyc-Cyc-CH₂CH₂-Phe-X-R² Ifg

R¹-Pyd-Phe-Z²-Phe-X-R² Ifh

R¹-Dio-Phe-Z²-Phe-X-R² Ifi

R¹-Phe-Cyc-Z²-Phe-X-R² Ifj

R¹-Phe-Cyc-Z²-Cyc-X-R² Ifk

R¹-Phe-Pyd-Z²-Phe-X-R² Ifl

R¹-Che-Phe-Z²-Phe-X-R² Ifm

R¹-Phe-Che-Z²-Phe-X-R² Ifn

R¹-Cyc-Phe-Z²-Phe-X-R² Ifo

R¹-Cyc-Phe-Z²-Cyc-X-R² Ifp

Darunter sind diejenigen der Formeln Ifc, Ife, Ifg und Ifo besonders bevorzugt, insbesondere worin Z² -CH₂CH₂-, -CO-O- oder -O-CO- bedeutet.

Die bevorzugten Verbindungen der Formeln Ig umfassen diejenigen der Formeln Iga bis Igf:

R¹-Phe-Phe-Phe-Phe-X-R² Iga

R¹-Cyc-Phe-Phe-Phe-X-R² Igb

R¹-Cyc-Cyc-Phe-Phe-X-R² Igc

R¹-Cyc-Cyc-Cyc-Phe-X-R² Igd

R¹-Cyc-Cyc-Cyc-Cyc-X-R² Ige

R¹-Cyc-Phe-Phe-Cyc-X-R² Iga

In den Verbindungen der vor- und nachstehenden Formeln bedeutet X vorzugsweise CF₂, CO-CF₂, O-CF₂, CHF-CF₂, CF₂-CHF oder O-CF₂-CHF bedeutet.

Insbesondere bevorzugt sind diejenigen, wobei X CF₂ bedeutet und eine der Gruppen Phe, vorzugsweise die direkt mit der Gruppe XR² verknüpfte Gruppe, einen Rest der Formel 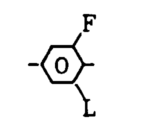 bedeutet, worin L H oder F ist.

In den Verbindungen der Formeln I, die eine terminale Gruppe -X-R² aufweisen, bedeutet R² vorzugsweise Alkyl mit 1 bis 12 C-Atomen, worin auch eine CH₂-Gruppe durch -0- ersetzt sein kann.

Somit bedeutet die endständige Gruppe X-R² vorzugsweise Gruppen die Formeln 1 bis 17

-CF₂-Alkyl 1

-Q-CF₂-Alkyl 2

-CHF-CF₂-Alkyl 3

-Q-CHF-CF₂-Alkyl 4

-CF₂-CHF-Alkyl 5

-Q-CF₂-CHF-Alkyl 6

-CHF-CHF₂ 7

-CF₂-CH₂-F 8

-Q-CHF-CHF₂ 9

-Q-CF₂-CH₂F 10

-CF₂-Q¹-alkyl 11

CH₂-CF₂-alkyl 13

CF₂-CF₂-alkyl 14

CH₂-CF₂-oxaalkyl 16

CF₂-CF₂-oxaalkyl 17

In den Gruppen 1 bis 6 bedeutet Alkyl vorzugsweise Alkyl oder Alkoxy mit 1 bis 8 C-Atomen.

In den Gruppen 2, 4 und 6 bedeutet Q vorzugsweise -O-, -CO- oder -O-CO-; in der Gruppe 11 bedeutet Q¹ -CO-, -CO-O-, -O-CO- oder -S-, insbesondere -CO- oder -S-.

R¹ bedeutet vorzugsweise Alkyl, ferner Alkoxy. R² bedeutet vorzugsweise Alkyl, Alkoxy, A¹, A², undloder A³ bedeuten bevorzugt Phe, Cyc, Che, Pyr oder Dio. Bevorzugt enthalten die Verbindungen der Formel I nicht mehr als einen der Reste Bi, Pyd, Pyr, Dio oder Dit.

Bevorzugt sind auch Verbindungen der Formel I sowie aller Teilformeln, in denen A¹, A², un/oder A³ ein- oder zweifach durch F oder einfach durch CN substituiertes 1,4-Phenylen bedeutet. Insbesondere sind dies 2-Fluor-1,4-phenylen, 3-Fluor-1,4-phenylen und 2,3-Difluor-1,4-phenylen sowie 2-Cyan-1,4-phenylen und 3-Cyan-1,4-phenylen.

Z¹ und Z² bedeuten bevorzugt eine Einfachbindung, -CO-O-, -O-CO- und -CH₂CH₂-, in zweiter Linie bevorzugt -CH₂O-und -OCH₂-.

Falls R¹ und/oder R² einen Alkylrest und/oder einen Alkoxyrest bedeutet, so kann dieser geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradecoxy.

Oxaalkyl bedeutet vorzugsweise geradkettiges 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

Falls R¹ und/oder R² einen Alkenylrest bedeuten, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-l-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5-oder Hept-6-enyl, Oct-1-, 2-, 3-, 4-, 5-, 6- oder Oct-7-enyl, Non-l-, 2-, 3-, 4-, 5-, 6-, 7- oder Non-8-enyl, Dec-1-, 2-, 3-, 4-, 5-, 6-, 7-, 8- oder Dec-9-enyl.

Falls R¹ und/oder R² einen Alkylrest bedeuten, in dem eine CH₂-Gruppe durch -0- und eine durch -CO- ersetzt ist, so sind diese bevorzugt benachbart. Somit beeinhalten diese eine Acyloxygruppe -CO-O- oder eine Oxycarbonylgruppe -O-CO-. Vorzugsweise sind diese geradkettig und haben 2 bis 6 C-Atome. Sie bedeuten demnach besonders Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxacarbonyl)ethyl, 2-(Propoxycarbonyl)ethyl, 3-(Methoxycarbonyl)propyl, 3-(Ethoxycarbonyl)propyl, 4-(Methoxycarbonyl)-butyl.

Falls R¹ undloder R² einen Alkenylrest bedeuten, in dem eine CH₂-Gruppe durch CO oder CO-O oder O-CO-ersetzt ist, so kann dieser geradkettig oder verzweigt sein. Vorzugsweise ist er geradkettig und hat 4 bis 13 C-Atome. Er bedeutet demnach besonders Acryloyloxymethyl, 2-Acryloyloxyethyl, 3-Acryloyloxypropyl, 4-Acryloyloxybutyl, 5-Acryloyloxypentyl, 6-Acryloyloxyhexyl, 7-Acryloyloxyheptyl, 8-Acryloyloxyoctyl, 9-Acryloyloxynonyl, 10-Acryloyloxydecyl, Methacryloyloxymethyl, 2-Methacryloyloxyethyl, 3-Methacryloyloxypropyl, 4-Methacryloyloxybutyl, 5-Methacryloyloxypentyl, 6-Methacryloyloxyhexyl, 7-Methacryloyloxyheptyl, 8-Methacryloyloxyoctyl, 9-Methacryloyloxynonyl.

Verbindungen der Formel I, die über für Polymerisationsreaktionen geeignete Flügelgruppen R¹ und/oder R² verfügen, eignen sich zur Darstellung flüssigkristalliner Polymere. Verbindungen der Formeln I mit verzweigten Flügelgruppen R¹ und/oder R² können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien.

Verbindungen der Formel I mit S_{A}-Phasen eignen sich beispielsweise für thermisch adressierte Displays.

Verzweigte Gruppen dieser Art enthalten in der Regel nicht mehr als eine Kettenverzweigung. Bevorzugte verzweigte Reste R¹ und/oder R² sind Isopropyl, 2-Butyl (= 1-Methylpropyl), Isobutyl (= 2-methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-l4ethylhexoxy, 1-Methylheptoxy, 2-Oxa-3-methylbutyl, 3-Oxa-4-methylpentyl, 4-Methylhexyl, 2-Nonyl, 2-Decyl, 2-Dodecyl, 6-Methyloctoxy, 6-Methyloctaroyloxy, 5-Methylheptyloxycarbonyl, 2-Methylbutyryloxy, 3-Methylvaleryloxy, 4-Methylhexanoyloxy, 2-Chlorpropionyloxy, 2-Chlor-3-methylbutyryloxy, 2-Chlor-4-methylvaleryloxy, 2-Chlor-3-methylvaleryloxy, 2-Methyl-3-oxapentyl, 2-Methyl-3-oxahexyl.

Falls R¹ oder R² einen Alkylrest darstellt, in dem zwei oder mehr CH₂-Gruppen durch -O- und/oder -CO-O- ersetzt sind, so kann dieser geradkettig oder verzweigt sein. Vorzugsweise ist er verzweigt und hat 3 bis 12 C-Atome. Er bedeutet demnach besonders Bis-carboxy-methyl, 2,2-Bis-carboxy-ethyl, 3,3-Bis-carboxy-propyl, 4,4-Bis-carboxy-butyl, 5,5-Bis-carboxy-pentyl, 6,6-Bis-carboxy-hexyl, 7,7-Bis-carboxy-heptyl, 8,8-Bis-carboxy-octyl, 9,9-Bis-carboxy-nonyl, 10,10-Bis-carboxy-decyl, Bis-(methoxycarbonyl)-methyl, 2,2-Bis-(methoxycarbonyl) -ethyl, 3,3-Bis-(methoxycarbonyl)-propyl, 4,4-Bis-(methoxycarbonyl)-butyl, 5,5-Bis-(methoxycarbonyl)-pentyl, 6,6-Bis-(methoxycarbonyl)-hexyl, 7,7-Bis-(methoxycarbonyl)-heptyl, 8,8-Bis-(methoxycarbonyl)-octyl, Bis-(ethoxycarbonyl)-methyl, 2,2-Bis-(ethoxycarbonyl)-ethyl, 3,3-Bis-(ethoxycarbonyl)-propyl, 4,4-Bis-(ethoxycarbonyl)-butyl, 5,5-Bis-(ethoxycarbonyl)-hexyl.

Verbindungen der Formel I, die über für Polykondensationen geeignete Flügelgruppen R¹ und/oder R² verfügen, eignen sich zur Darstellung flüssigkristalliner Polykondensate.

Formel I umfaßt sowohl die Racemate dieser Verbindungen als auch die optischen Antipoden sowie deren Gemische.

Unter diesen Verbindungen der Formel I sowie den Unterformeln sind diejenigen bevorzugt, in denen mindestens einer der darin enthaltenden Reste eine der angegebenen bevorzugten Bedeutungen hat.

In den Verbindungen der Formel I sind diejenigen Stereoisomeren bevorzugt, in denen die Ringe Cyc und Piperidin trans-1,4-disubstituiert sind. Diejenigen der vorstehend genannten Formeln, die eine oder mehrere Gruppen Pyd, Pyr und/oder Dio enthalten, umschließen jeweils die beiden 2,5-Stellungsisomeren.

Insbesonders bevorzugte Verbindungen der Formeln I mit den Flügelgruppen der Formeln 1 sind die der Teilformeln Ila bis Ily:

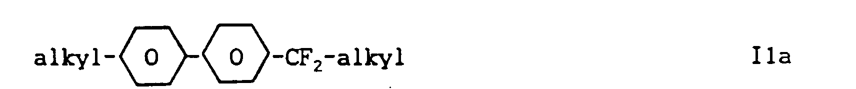

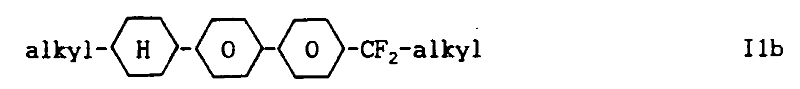

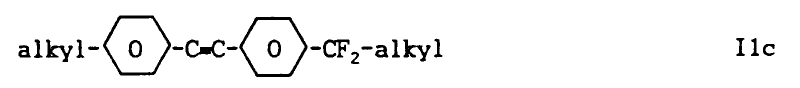

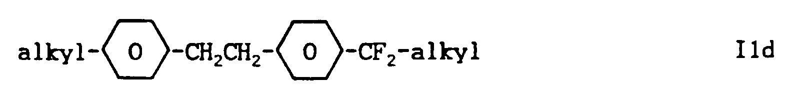

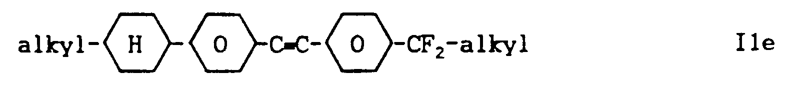

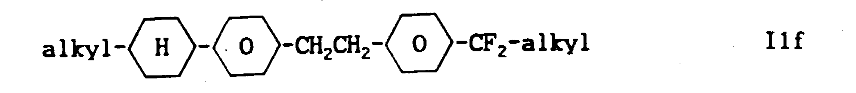

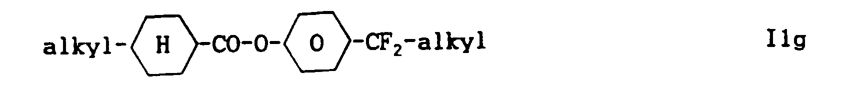

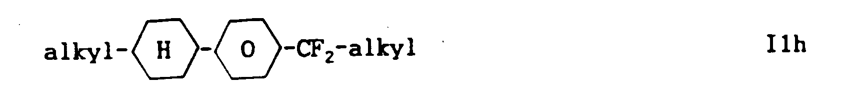

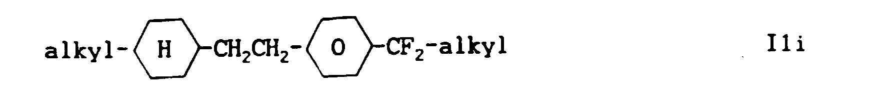

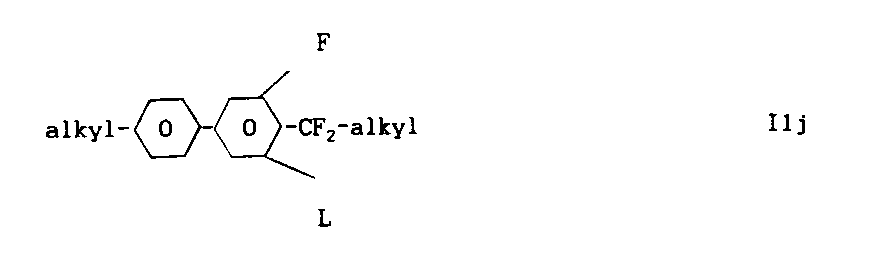

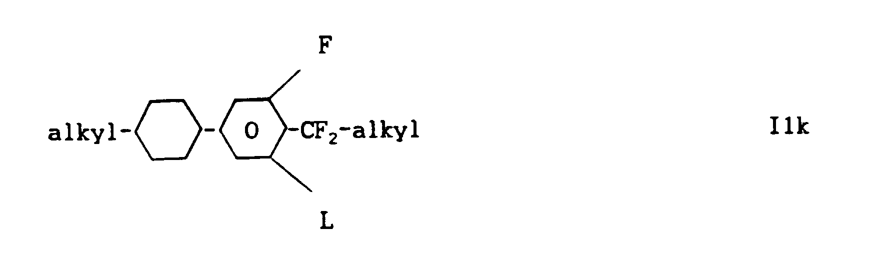

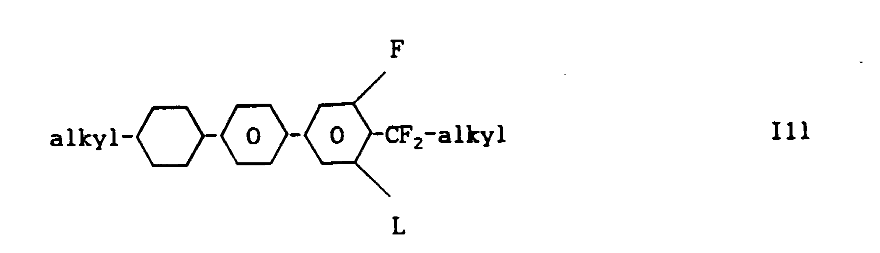

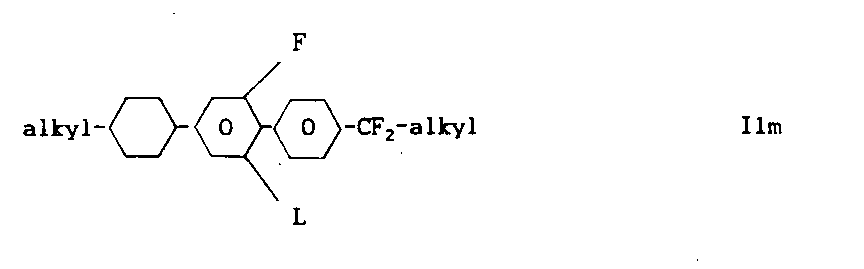

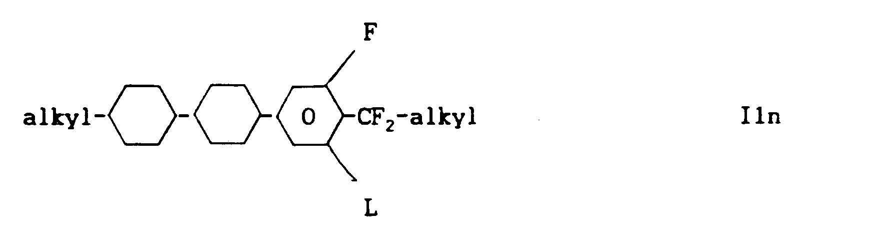

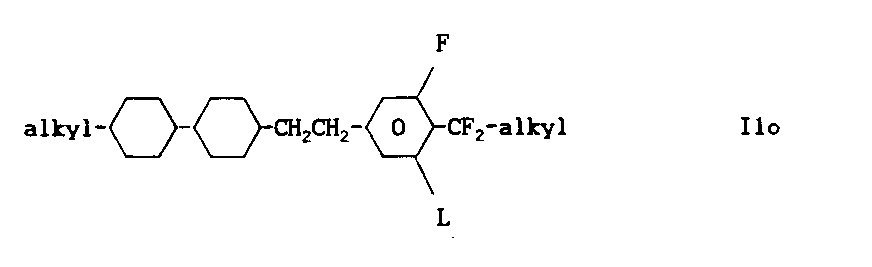

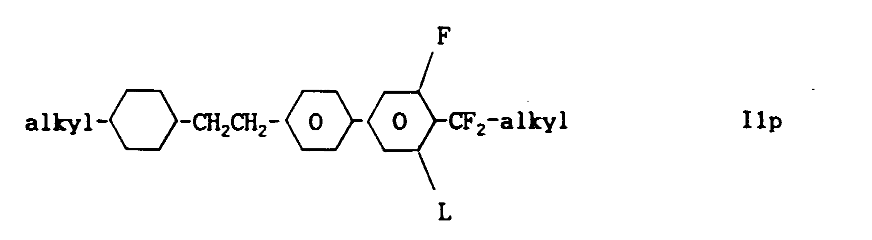

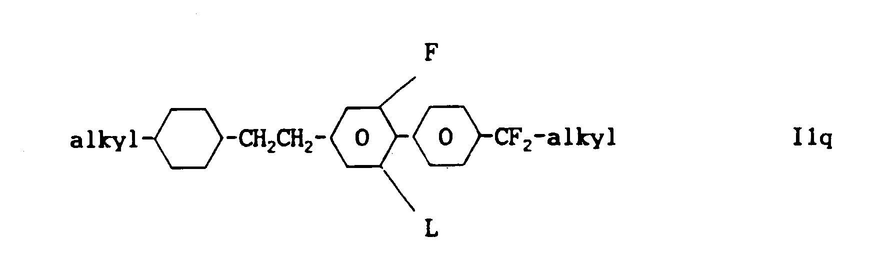

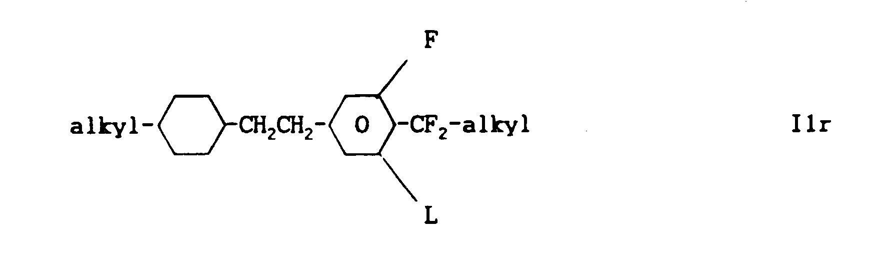

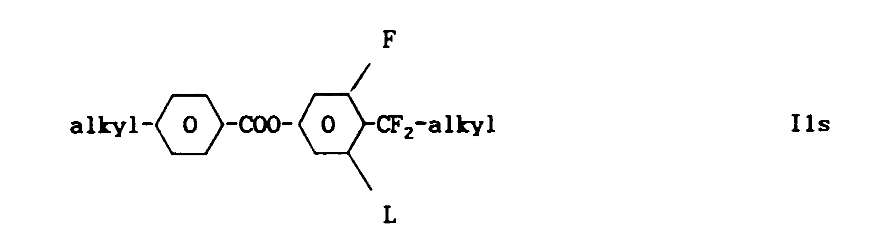

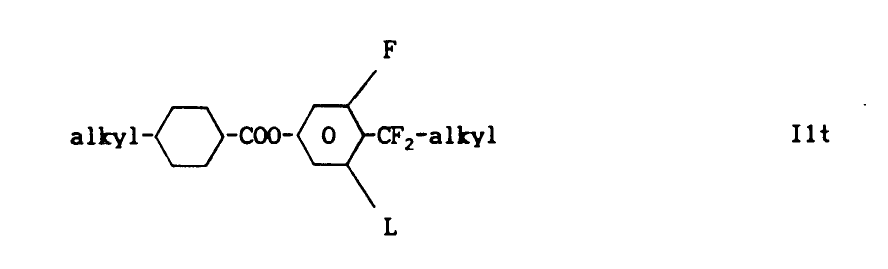

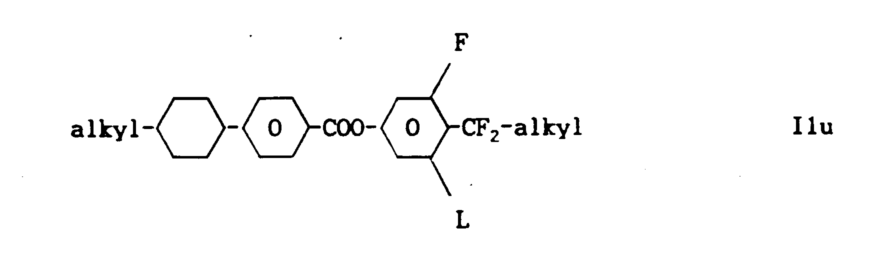

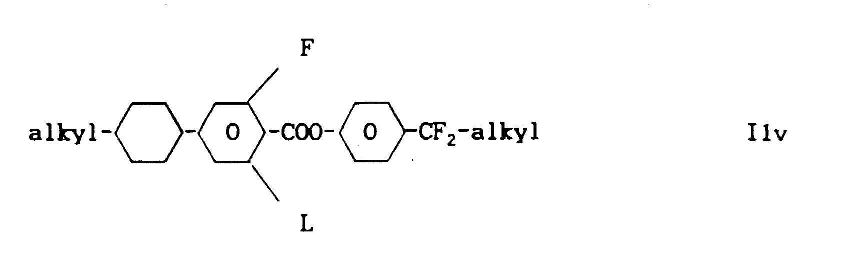

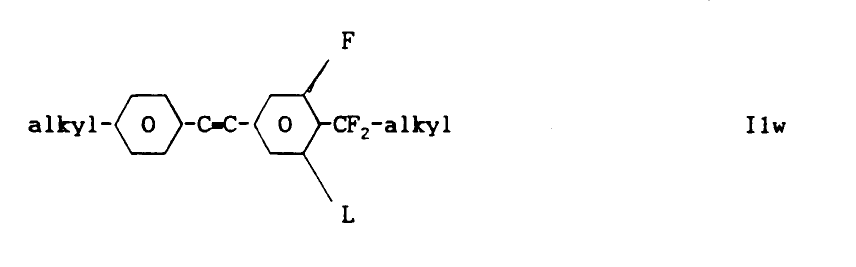

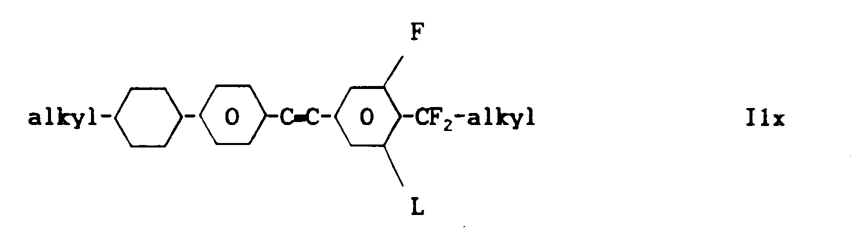

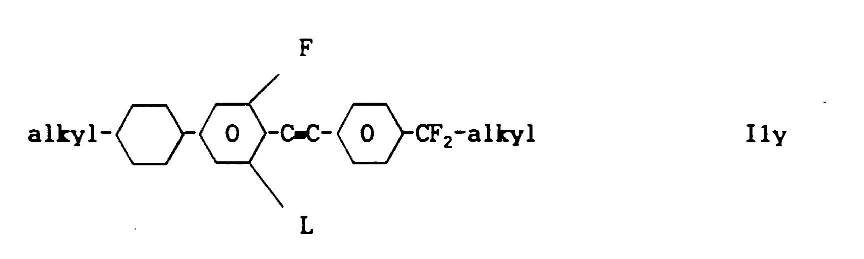

Insbesondere bevorzugte Verbindungen der Formeln I mit den Flügelgruppen der Formeln 2 sind diejenigen der Teilformeln I2a bis I2i:

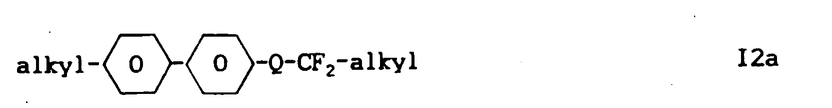

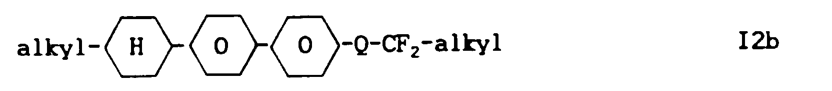

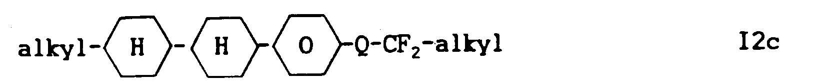

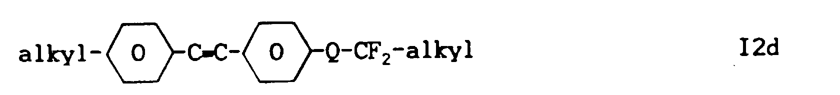

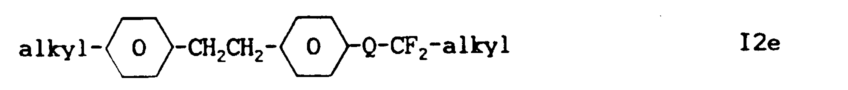

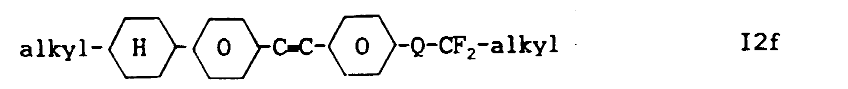

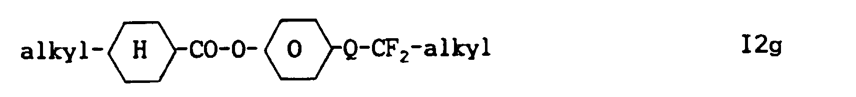

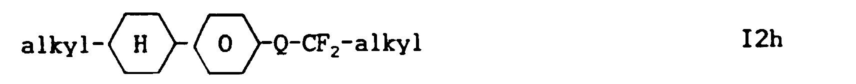

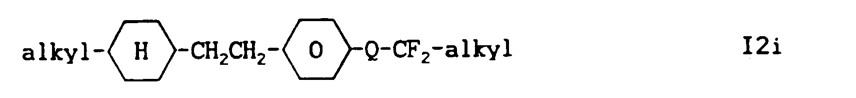

Insbesondere bevorzugte Verbindungen der Formel I mit den Flügelgruppen der Formeln 3 sind diejenigen der Teilformeln I3a bis I3g:

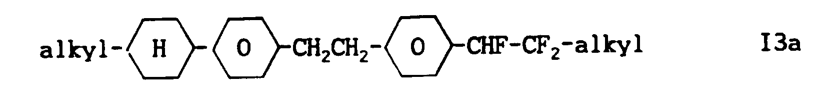

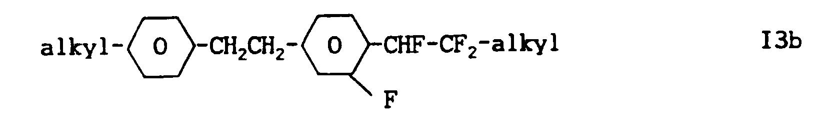

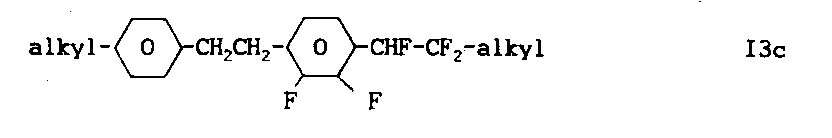

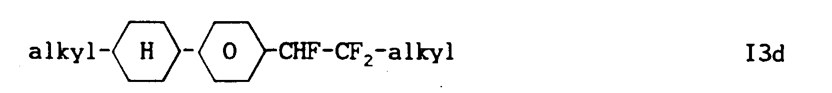

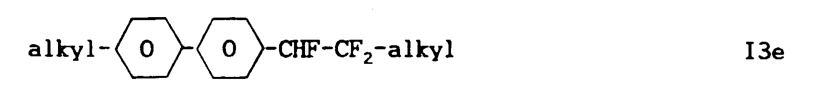

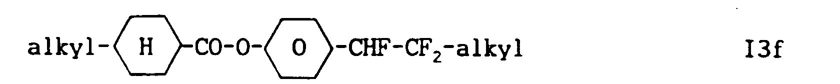

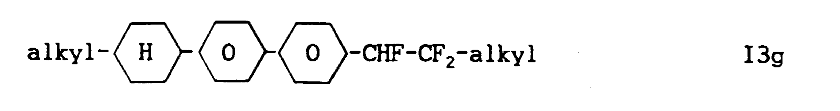

Insbesondere bevorzugte Verbindungen der Formeln I mit den Flügelgruppen der Formeln 4 sind diejenigen der Teilformeln I4a bis I4j:

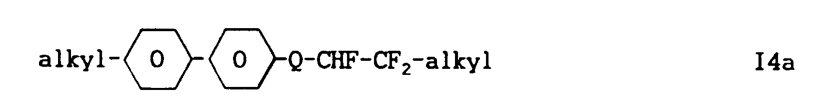

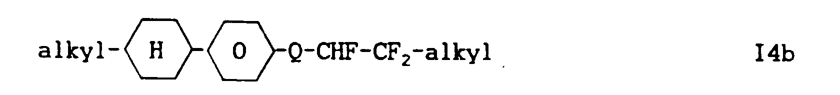

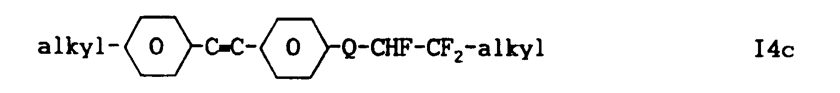

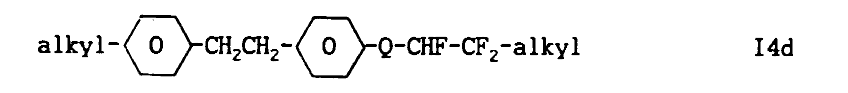

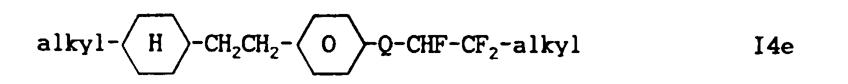

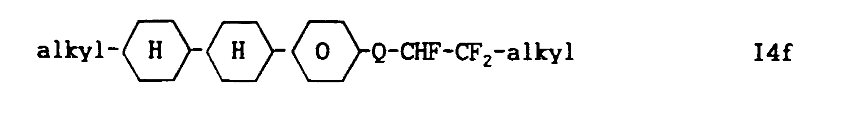

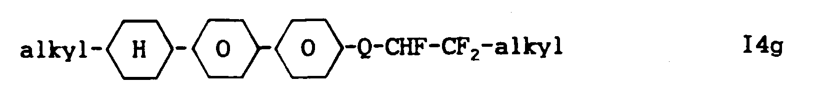

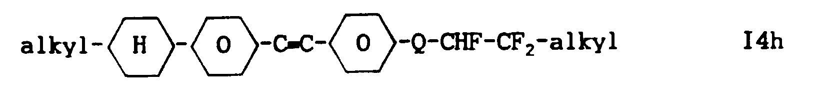

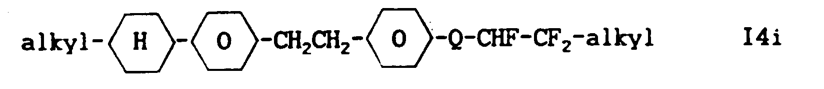

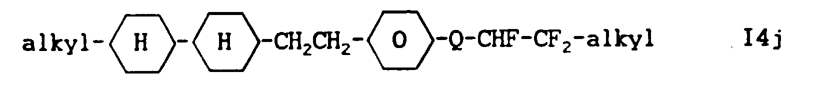

Insbesondere bevorzugte Verbindungen der Formeln I mit den Flügelgruppen der Formeln 5 sind diejenigen der Teilformeln I5a bis I5j:

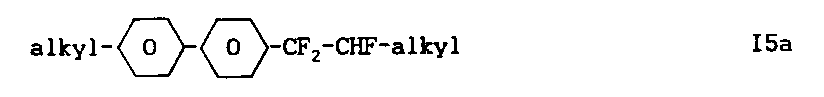

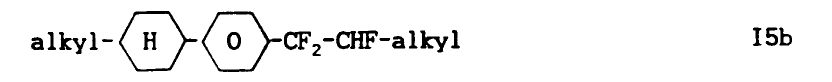

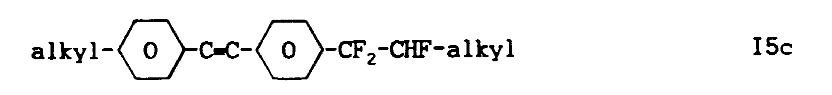

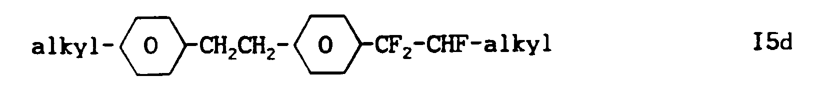

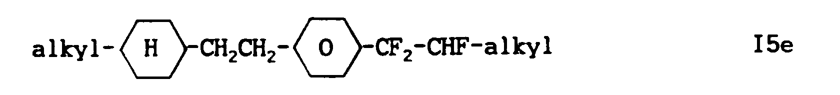

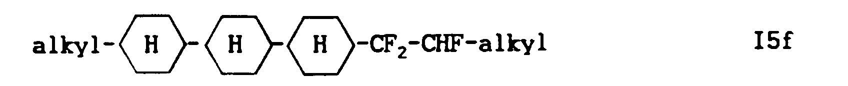

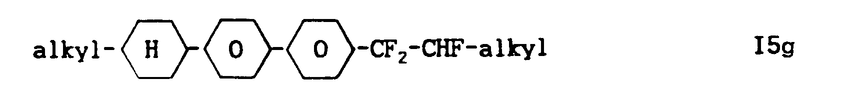

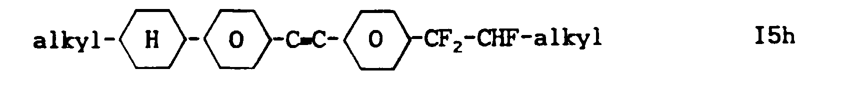

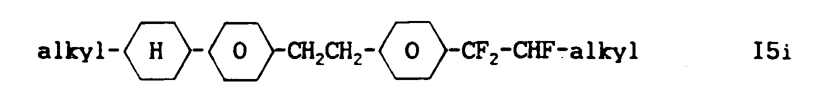

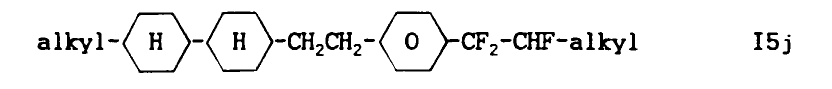

Insbesondere bevorzugte Verbindungen der Formeln I mit den Flügelgruppen der Formeln 6 sind diejenigen der Teilformeln I6a bis I6j:

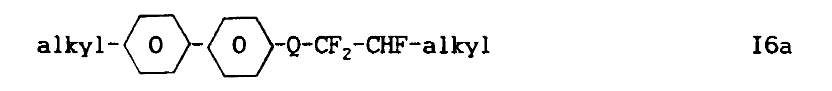

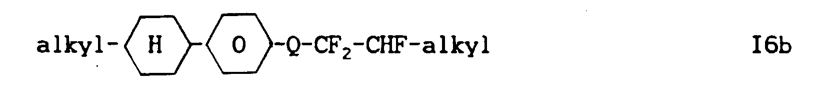

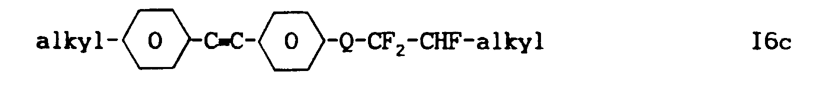

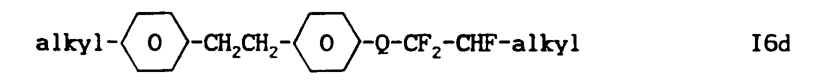

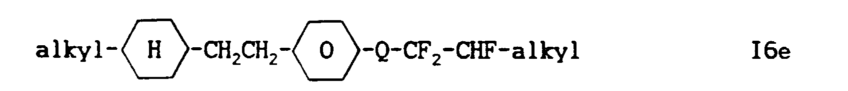

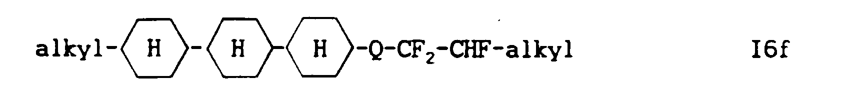

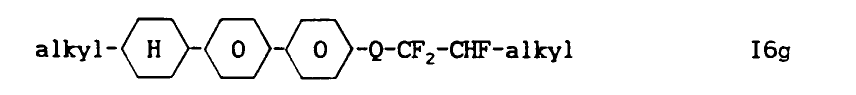

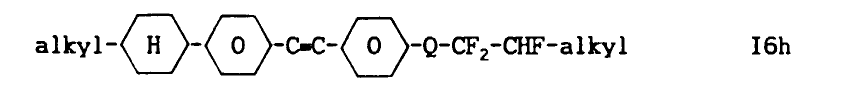

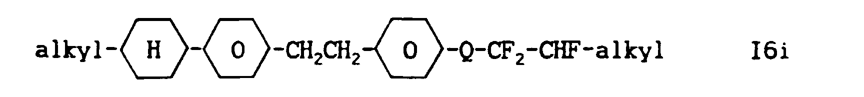

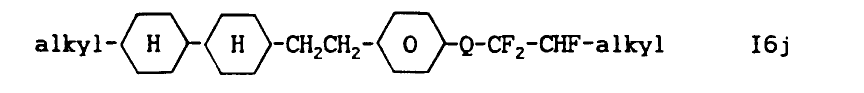

Insbesondere bevorzugte Verbindungen der Formeln I mit den Flügelgruppen der Formeln 11 sind diejenigen der Teilformeln I11a bis I11k:

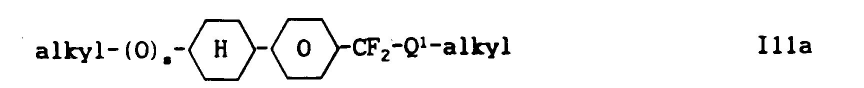

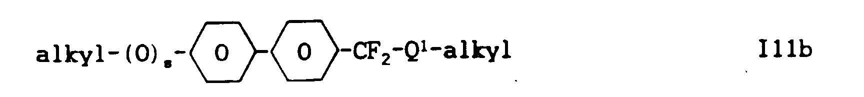

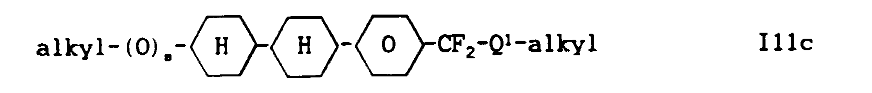

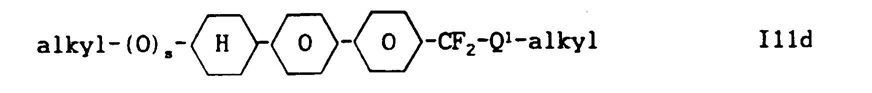

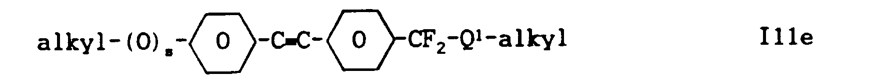

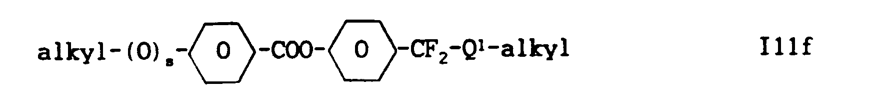

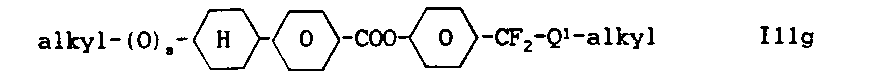

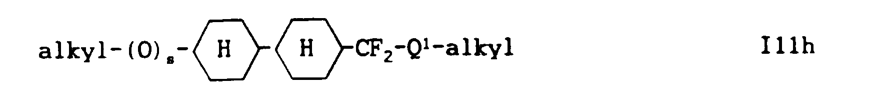

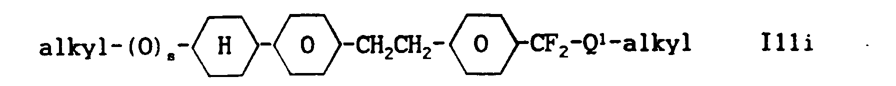

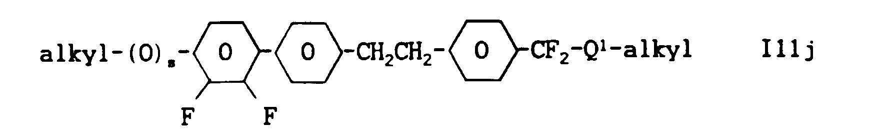

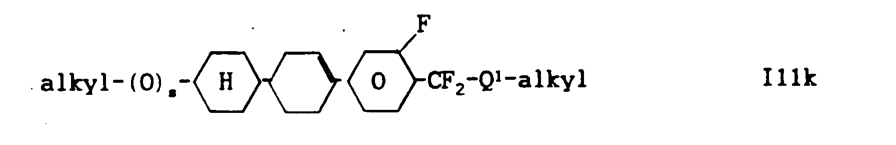

Insbesondere bevorzugte Verbindungen der Formel I, welche eine Gruppe der Formel 13 aufweisen, sind diejenigen der - Teilformeln I13a bis I13n:

alkyl-Phe-Phe-CH₂CF₂-alkyl I12a

alkyl-Cyc-Phe-CH₂CF₂-alkyl I13b

alkyl-Cyc-Phe-Phe-CH₂-CF₂-alkyl I13c

alkyl-Cyc-Cyc-Phe-CH₂-CF₂-alkyl I13d

alkyl-Phe-Phe-Phe-CH₂-CF₂-alkyl I13e

alkyl-Phe-CH₂CH₂-Phe-CH₂-CF₂-alkyl I13f

alkyl-Cyc-CH₂CH₂-Phe-CH₂-CF₂-alkyl I13g

alkyl-Cyc-CO-O-Phe-CH₂-CF₂-alkyl I13h

alkyl-Phe-CO-O-Phe-CH₂-CF₂-alkyl I13i

alkyl-Cyc-Phe-CC-Phe-CH₂-CF₂-alkyl I13j

alkyl-Cyc-Phe-CH₂CH₂-Phe-CH₂-CF₂-alkyl I13k

alkyl-Cyc-Cyc-CH₂CH₂-Phe-CH₂-CF₂-alkyl I13l

alkyl-Phe-Phe-CH₂CH₂-Phe-CH₂-CF₂-alkyl I13m

alkyl-Cyc-CH₂CH₂-Cyc-Phe-CH₂-CF₂-alkyl I13n

Insbesondere bevorzugte Verbindungen der Formel I, welche eine Gruppe der Formel 14 aufweisen, sind diejenigen der Formeln I14a bis I14n:

alkyl-Phe-Phe-CF₂-CF₂-alkyl I14a

alkyl-Cyc-Phe-CF₂-CF₂-alkyl I14b

alkyl-Cyc-Phe-Phe-CF₂-CF₂-alkyl I14c

alkyl-Cyc-Cyc-Phe-CF₂-CF₂-alkyl I14d

alkyl-Phe-Phe-Phe-CF₂-CF₂-alkyl I14e

alkyl-Phe-CH₂CH₂-Phe-CF₂-CF₂-alkyl I14f

alkyl-Cyc-CH₂CH₂-Phe-CF₂-CF₂-alkyl I14g

alkyl-Cyc-CO-O-Phe-CF₂-CF₂-alkyl I14h

alkyl-Phe-CO-O-Phe-CF₂-CF₂-alkyl I14i

alkyl-Cyc-Phe-CC-Phe-CF₂-CF₂-alkyl I14j

alkyl-Cyc-Phe-CH₂CH₂-Phe-CF₂-CF₂-alkyl I14k

alkyl-Cyc-Cyc-CH₂CH₂-Phe-CF₂-CF₂-alkyl I14l

alkyl-Phe-Phe-CH₂CH₂-Phe-CF₂-CF₂-alkyl I14m

alkyl-Cyc-CH₂CH₂-Cyc-Phe-CF₂-CF₂-alkyl I14n

In den voranstehenden Verbindungen der Teilformeln Ila bis Ily, I2a bis I2i, I3a bis I3g und I4a bis I4j, I5d bis I5j, I6a bis I6j, I12a bis I12m, I13a bis I13n und I14a bis I14n bedeuten alkyl- jeweils Alkyl- bzw. Alkoxygruppen mit 1 bis 12 C-Atomen.

In den voranstehenden Verbindungen der Formeln Illa bis Illk bedeuten alkyl jeweils Alkylgruppen mit 1 bis 12 C-Atomen, O oder 1 und Q¹ -CO-, -O-CO-, -CO-O- oder -S-.

Die 1,4-Cyclohexenylen-Gruppe hat vorzugsweise folgende Strukturen:

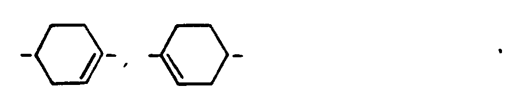

Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart Bd IX, S. 867 ff.) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Verbindungen der Formel I, worin -(Y)ₙ-Q- -CO-CF₂ bedeutet, können durch Umsetzung von Metallorganischen Verbindungen der Formel R¹-A¹-Z¹-(A²-Z²)-A³-Met, worin Met vorzugsweise Li, Na, K, MgBr, MgCl oder ZnBr, insbesondere Li, bedeutet, mit 2,2-Difluoralkansäurealkylestern hergestellt werden. Aus diesen erhält man durch Reduktion mit komplexen Hydriden wie z. B. Natriumborhydrid, Lithiumborhydrid, Diisobutylaluminiumhydrid oder Lithiumaluminiumhydrid und anschließende Fluorierung mit einem Dialkylaminoschwefelfluorid, beispielsweise DAST (Diethylaminoschwefeltrifluorid), die Verbindungen der Formel I, worin -(Y)ₙ-Q-CHF-CF₂ bedeutet (vgl. Schema I). 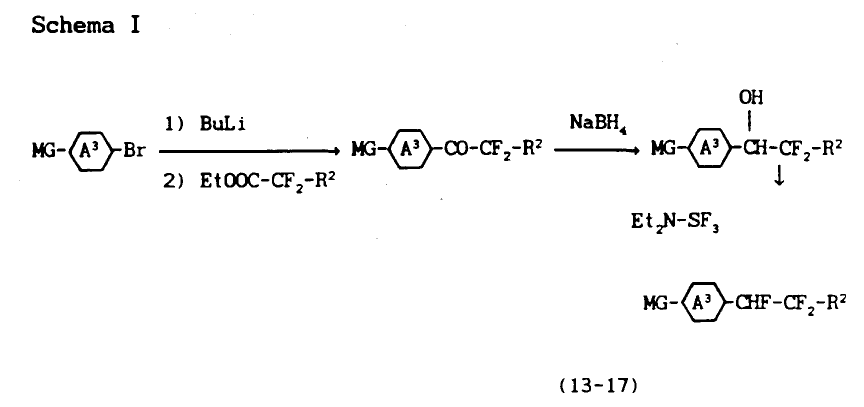 MG bedeutet vor- und nachstehend eine mesogene Gruppe der Formel R¹-A¹-Z¹-(A²Z²-)ₘ.

Die 2,2-Difluoralkanoylarylverbindungen der Formel I, wobei (Y)ₙ-Q = CO-CF₂ und A³ ein gegebenenfalls substituierter 1,4-Phenylenrest bedeutet, worin auch eine oder zwei CH-Gruppen durch N ersetzt sein können, können auch hergestellt werden, indem man die entsprechende Arylverbindung, worin (Y)ₙ-Q-R² H bedeutet, z. B. mit 2,2-Difluoracylchloriden unter Friedel-Crafts-Bedingungen miteinander umsetzt [z. B. J.H. Simmons, E.O. Ramler J.Am.Chem. Soc. 65 (1943), 389].

Die Verbindungen der Formel I, worin n = 0 ist und Q CF₂ bedeutet, können aus den entsprechenden Ketonen,worin (Y)ₙ-Q CO bedeutet, nach Überführung in die entsprechenden Thioketale durch Umsetzen mit einem Bromierungsmittel wie z. B. 1,3-Dibrom-5,5-dimethylhydantoin (NDBDH) oder N-Bromsuccinimid (NBS) und einem Fluorierungsmittel wie z. B. Pyridiniumfluorid, Tetrabutylammoniumfluorid, DAST oder Cäsiumfluorid, z. B. gemäß Schema II hergestellt werden.

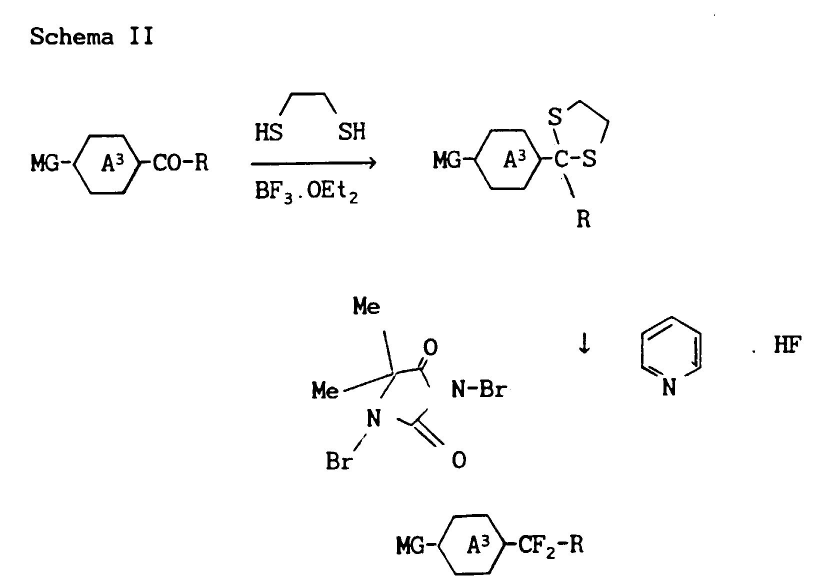

Die Verbindungen der Formel I, worin n = 0 ist und Q CF₂-CHF bedeutet, lassen sich durch Umsetzung der aus den entsprechenden Formylverbindung (worin (Y)ₙ-Q-R²CO-H bedeutet) erhältlichen Thioacetale durch Deprotonierung und Umsetzung mit Aldehyden der Formel R²-CHO, anschließende Fluorierung der so erhaltenen Hydroxygruppe mit z. B. DAST und schließlich durch Umsetzung mit einem Bromierungsmittel und einen Fluorierungsmittel gemäß Schema III erhalten

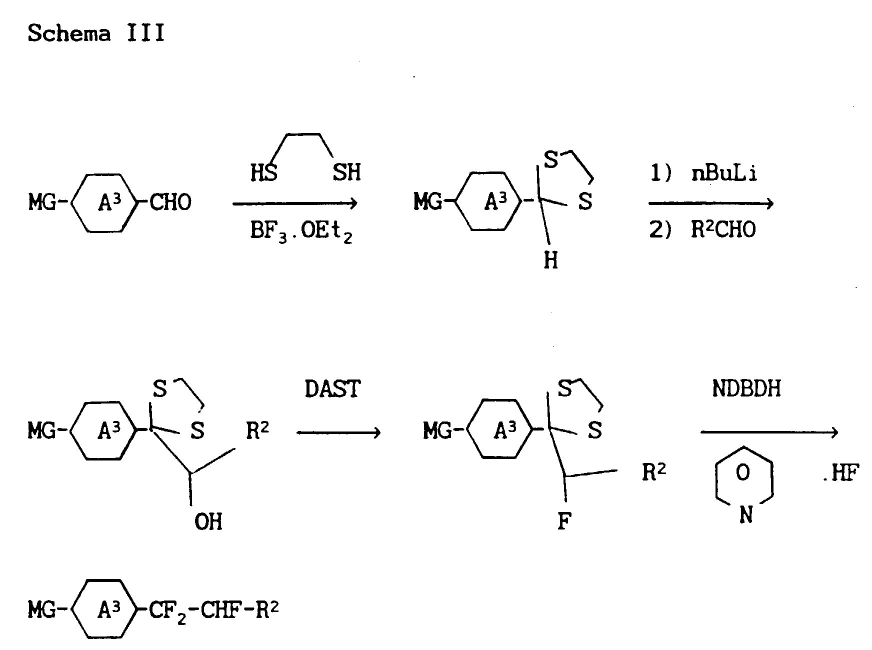

Die Verbindungen der Formel I, worin Y -COO- oder -O-bedeutet, lassen sich z. B. gemäß Schema IV herstellen.

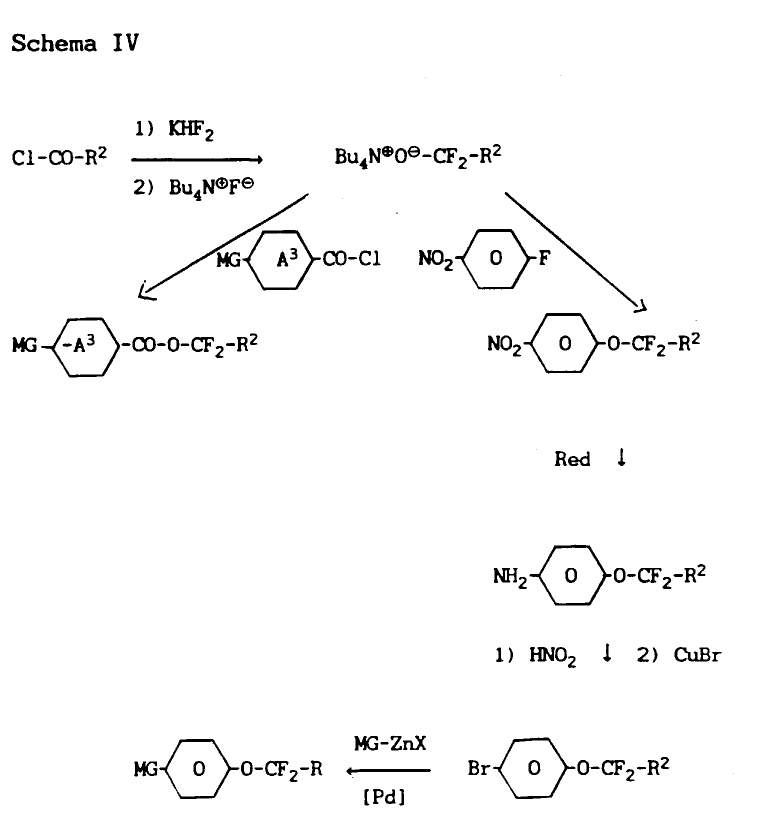

Die Verbindungen der Formel I' (worin A³ 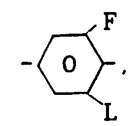 n 0 und Q CF₂ bedeutet) können z.B. entsprechend Schema V hergestellt werden.

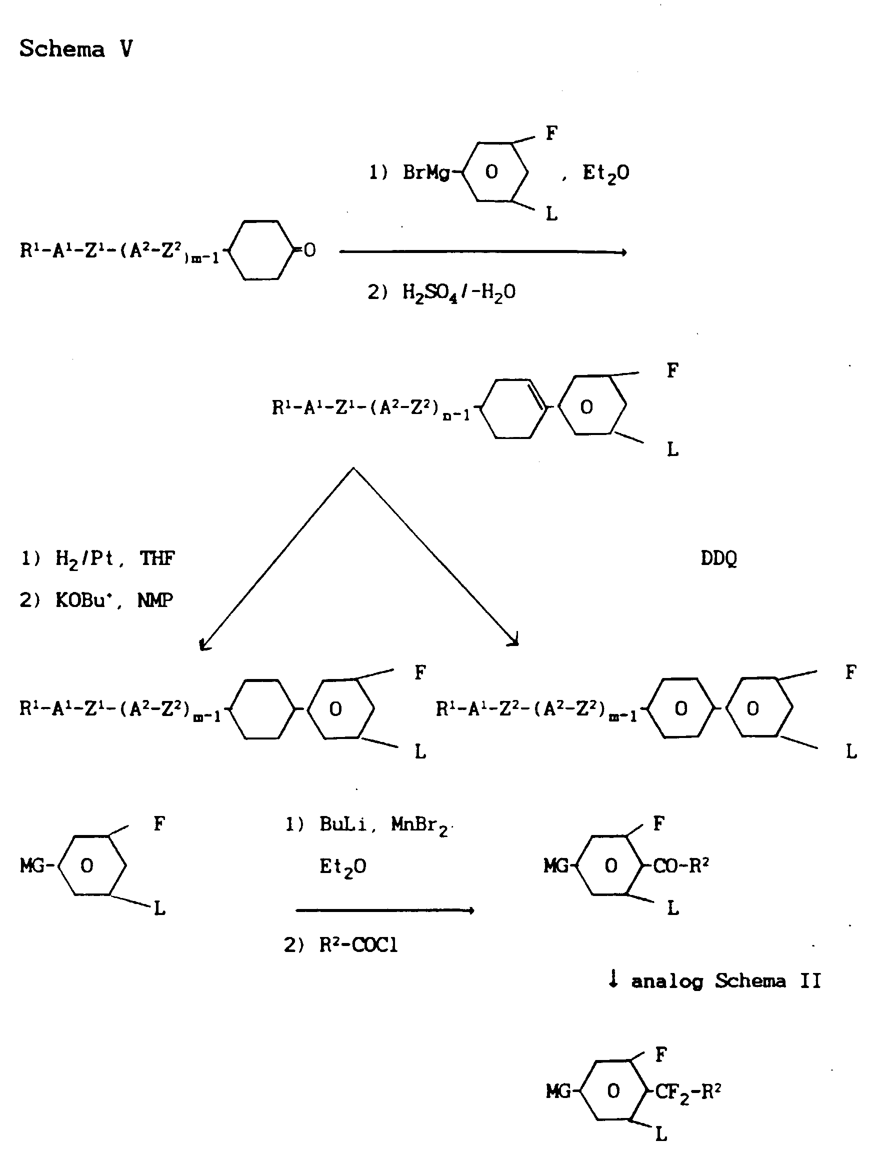

Die Verbindungen der Formel I mit den Flügelgruppen der Formel 11, worin Q¹ -CO-O- oder CO bedeutet, können entsprechend Schema VI oder VII hergestellt werden: 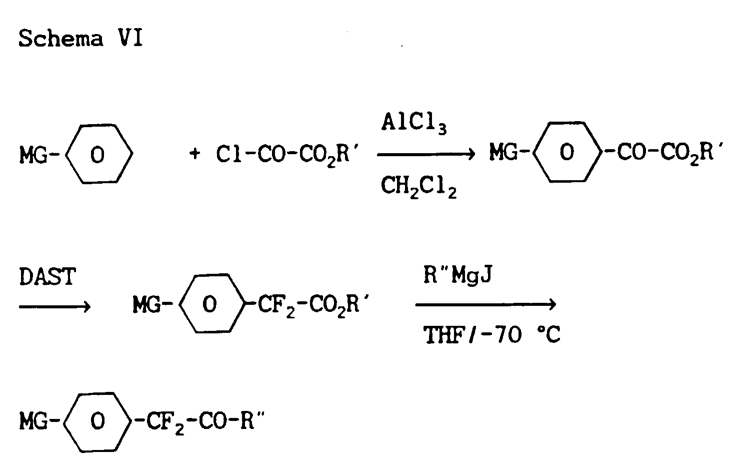
- R': n-Alkyl mit 1 bis 12 C-Atomen
- R": n-Alkyl mit 1 bis 12 C-Atomen

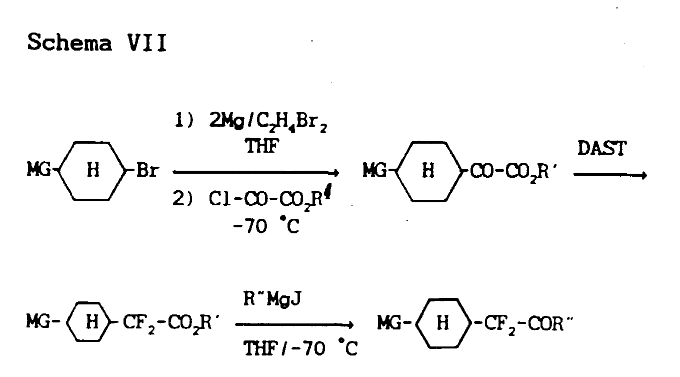

Die Verbindungen der Formel I, mit den Flügelgruppen der Formel 11, worin Q¹ -S- oder -O-CO- bedeutet, bzw. mit den Flügelgruppen der Formel 1, worin CF₂ mit einer Alkoxygruppe verknüpft ist, lassen sich gemäß Schema VIII herstellen:

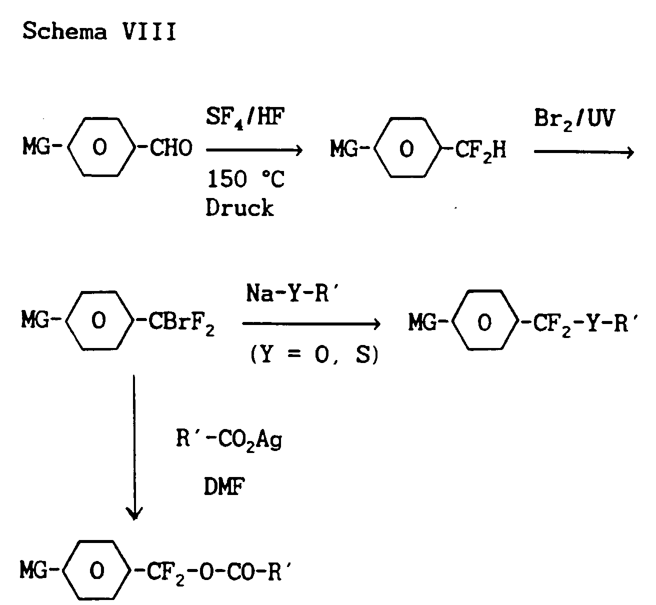

Verbindungen der Formel I, worin -(Y)ₙ-Q- -CH₂-CF₂- bedeutet, können durch Umsetzung von metallorganischen Verbindungen der Formel R¹-A¹-Z¹-(A²-Z²)ₘ-A³-Met, worin Met vorzugsweise Li, Na, K, MgBr, MgCl oder ZnBr, insbesondere Li bedeutet, mit Epoxiden hergestellt werden. Aus den so hergestellten Hydroxyverbindungen erhält man nach Oxidaton mit z.B. Pyridin im Chlorochromat, durch Fluorierung mit einem Dialkylaminoschwefeltrifluorid, beispielsweise DAST, die entsprechenden Verbindungen der Formel I (vgl. Schema IX):

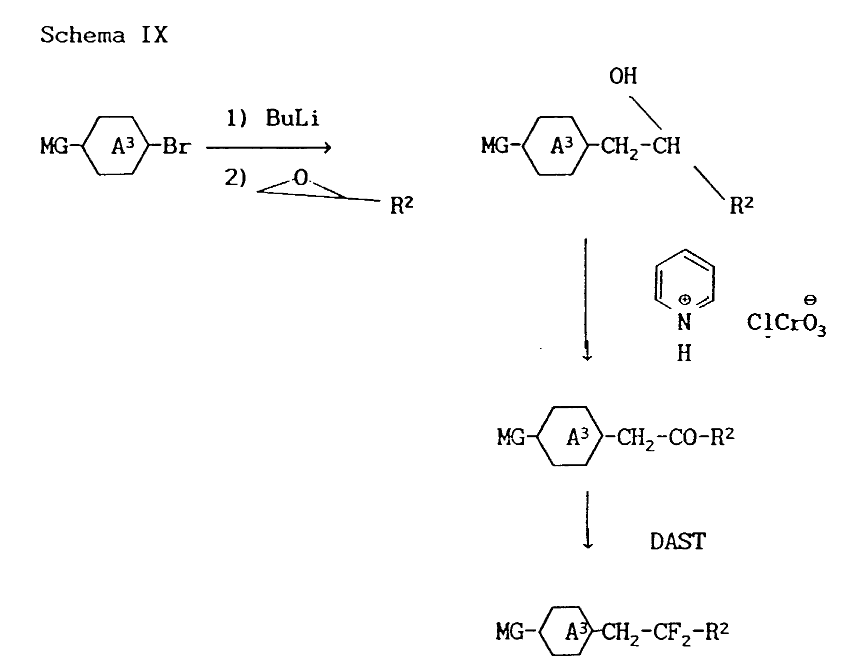

- MG: bedeutet voranstehend und im folgenden eine der Formel R¹-A¹-Z¹-(A²-Z²)ₘ entsprechende mesogene Gruppe.

Verbindungen der Formel I, worin (Y)ₙ-Q -CF₂-CF₂- bedeutet, können durch Umsetzen von metallorganischen Verbindungen der Formel MG-A³-Met mit 2,2-Difluoralkansäurealkylester und anschließender Fluorierung gemäß Schema X hergestellt werden:

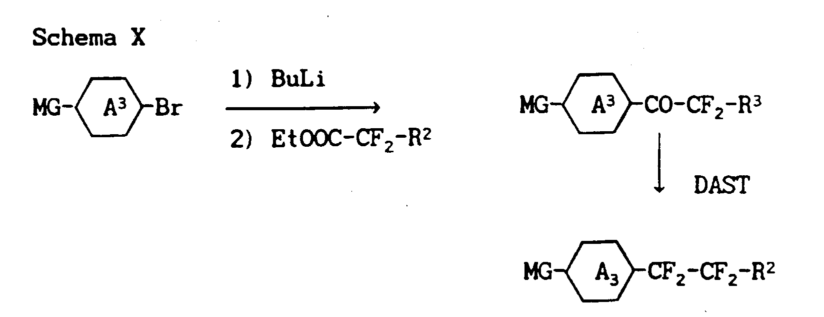

Verbindungen der Formel I, worin (Y)ₙ-Q CF₂-CHF- bedeutet, können nach Überführung der entsprechenden Formylverbindungen der Formel MG- A³ -CHO in die Thioacetale, durch Deprotonierung und Umsetzung mit Aldehyden der Formel R²-CHO, anschließende Fluorierung der so erhaltenen Hydroxygruppe mit z.B. DAST und anschließende Überführung der Thioketale durch Umsetzung mit einem Bromierungsmittel wie z.B. 1,3-Dibrom-5,5-dimethylhydantoin (NDBDH) oder N-Bromsuccinimid (NBS) in Gegenwart eines Fluorierungsmittels wie z.B. Pyridiniumfluorid, Tetrabutylammoniumfluorid, DAST, Xenondifluorid oder Cäsiumfluorid, z.B. nach Schema XI hergestellt werden:

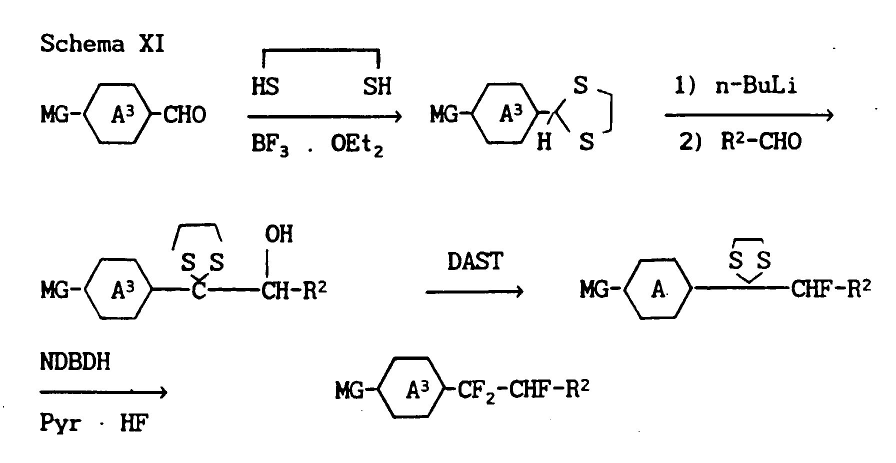

Ausgangstoffe können gewünschtenfalls auch in situ gebildet werden, derart, daß man sie aus dem Reaktionsgemisch nicht isoliert, sondern sofort weiter zu den Verbindungen der Formel I umsetzt.

So können die Verbindungen der Formel I hergestellt werden, indem man eine Verbindung, die sonst der Formel I entspricht, aber an Stelle von H-Atomen eine oder mehrere reduzierbare Gruppen und/oder C-C-Bindungen enthält, reduziert.

Als reduzierbare Gruppen kommen vorzugsweise Carbonylgruppen in Betracht, insbesondere Ketogruppen, ferner z.B. freie oder veresterte Hydroxygruppen oder aromatisch gebundene Halogenatome. Bevorzugte Ausgangsstoffe für die Reduktion entsprechen der Formel I, können aber an Stelle eines Cyclohexanringes einen Cyclohexenring oder Cyclohexanonring und/oder an Stelle einer -CH₂CH₂-Gruppe eine -CH=CH-Gruppe und/oder an Stelle einer -CH₂-Gruppe eine -CO-Gruppe und/oder an Stelle eines H-Atoms eine freie oder eine funktionell (z.B. in Form ihres p-Toluolsulfonats) abgewandelte OH-Gruppe enthalten.

Die Reduktion kann z.B. erfolgen durch katalytische Hydrierung bei Temperaturen zwischen etwa 0° und etwa 200° sowie Drucken zwischen etwa 1 und 200 bar in einem inerten Lösungsmittel, z.B. einem Alkohol wie Methanol, Ethanol oder Isopropanol, einem Ether wie Tetrahydrofuran (THF) oder Dioxan, einem Ester wie Ethylacetat, einer Carbonsäure wie . Essigsäure oder einem Kohlenwasserstoff wie Cyclohexan. Als Katalysatoren eignen sich zweckmäßig Edelmetalle wie Pt oder Pd, die in Form von Oxiden (z.B. PtO₂, PdO), auf einem Träger (z.B. Pd auf Kohle, Calciumcarbonat oder Strontiumcarbonat) oder in feinverteilter Form eingesetzt werden können.

Ketone können auch nach den Methoden von Clemmensen (mit Zink, amalgamiertem Zink oder Zinn und Salzsäure, zweckmäßig in wäßrig-alkoholischer Lösung oder in heterogener Phase mit Wasser/Toluol bei Temperaturen zwischen etwa 80 und 120°) oder Wolff-Kishner (mit Hydrazin, zweckmäßig in Gegenwart von Alkali wie KOH oder NaOH in einem hochsiedenden Lösungsmittel wie Diethylenglykol oder Triethylenglykol bei Temperaturen zwischen etwa 100 und 200°) zu den entsprechenden Verbindungen der Formel I, die Alkylgruppen und/oder -CH₂CH₂-Brücken enthalten, reduziert werden.

Weiterhin sind Reduktionen mit komplexen Hydriden möglich. Beispielsweise können Arylsulfonyloxygruppen mit LiAlH₄ reduktiv entfernt werden, insbesondere p-Toluolsulfonyloxymethylgruppen zu Methylgruppen reduziert werden, zweckmäßig in einem inerten Lösungsmittel wie Diethylether oder THF bei Temperaturen zwischen etwa 0 und 100°. Doppelbindungen können mit NaBH₄ oder Tributylzinnhydrid in Methanol hydriert werden.

Verbindungen der Formel I, die ansonsten der Formel I entsprechen, aber an Stelle von 1,4-Phenylenresten 1,4-Cyclohexenylenreste besitzen, können zum Beispiel mit DDQ (Dichlordicyanobenzochinon) in einem geeigneten Lösungsmittel oxidiert werden.

Ester der Formel I können auch durch Veresterung entsprechender Carbonsäuren (oder ihrer reaktionsfähigen Derivate) mit Alkoholen bzw. Phenolen (oder ihren reaktionsfähigen Derivaten) oder nach der DCC-Methode (DCC = Dicyclohexylcarbodiimid) erhalten werden.

Die entsprechenden Carbonsäuren und Alkohole bzw. Phenole sind bekannt oder können in Analogie zu bekannten Verfahren hergestellt werden.

Als reaktionsfähige Derivate der genannten Carbonsäuren eignen sich insbesondere die Säurehalogenide, vor allem die Chloride und Bromide, ferner die Anhydride, z.B. auch gemischte Anhydride, Azide oder Ester, insbesondere Alkylester mit 1-4 C-Atomen in der Alkylgruppe.

Als reaktionsfähige Derivate der genannten Alkohole bzw. Phenole kommen insbesondere die entsprechenden Metall-alkoholate bzw. Phenolate, vorzugsweise eines Alkalimetalls wie Natrium oder Kalium, in Betracht.

Die Veresterung wird vorteilhaft in Gegenwart eines inerten Lösungsmittels durchgeführt. Gut geeignet sind insbesondere Ether wie z.B. Diethylether, Di-n-butylether, THF, Dioxan oder Anisol, Ketone wie z. B. Aceton, Butanon oder Cyclohexanon, Amide wie z. B. DMF oder Phosanon, Amide wie z. B. DMF oder Phosphorsäurehexamethyltriamid, Kohlenwasserstoffe wie z.B. Benzol, Toluol oder Xylol, Halogenkohlenwasserstoffe wie z. B. Tetrachlorkohlenstoff, Dichlormethan oder Tetrachlorethylen und Sulfoxide wie z. B. Dimethylsulfoxid oder Sulfolan.

Zur Herstellung von Nitrilen der Formel I können entsprechende Säureamide, z.B. solche, in denen an Stelle des Restes CN eine CONH₂-Gruppe steht, dehydratisiert werden. Die Amide sind z.B. aus entsprechenden Estern oder Säurehalogeniden durch Umsetzung mit Ammoniak erhältlich. Als wasserabspaltende Mittel eignen sich beispielsweise anorganische Säurechloride wie SOCl₂, PCl₃, PCl₅, POCl₃, SO₂Cl₂, COCl₂, ferner P₂O₅, P₂S₅, AlCl₃ (z.B. als Doppelverbindung mit NaCl), aromatische Sulfonsäuren und Sulfonsäurehalogenide. Man kann dabei in Gegenwart oder Abwesenheit eines inerten Lösungsmittels bei Temperaturen zwischen etwa 0° und 150° arbeiten; als Lösungsmittel kommen z.B. Basen wie Pyridin oder Triethylamin, aromatische Kohlenwasserstoffe wie Benzol, Toluol oder Xylol oder Amide wie DMF in Betracht.

Zur Herstellung der vorstehend genannten Nitrile der Formel I kann man auch entsprechende Säurehalogenide, vorzugsweise die Chloride, mit Sulfamid umsetzen, zweckmäßig in einem inerten Lösungsmittel wie z. B. Tetramethylensulfon bei Temperaturen zwischen etwa 80° und 150°, vorzugsweise bei 120°. Nach üblicher Aufarbeitung kann man direkt die Nitrile isolieren.

Ether der Formel I sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z.B. durch Behandeln mit NaH, NaNH₂, NaOH, KOH, Na₂CO₃ oder K₂CO₃ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie z. B. Aceton, 1,2-Dimethoxyethan, DMF oder Dimethylsulfoxid oder auch mit einem Überschuß an wäßriger oder wäßrig-alkoholischer NaOH oder KOH bei Temperaturen zwischen etwa 20° und 100°.

Zur Herstellung von Nitrilen der Formel I können auch entsprechende Chlor-, Brom- oder Jodverbindungen der Formel I mit einem Cyanid umgesetzt werden, vorzugsweise mit einem Metallcyanid wie z. B. NaCN, KCN oder Cu₂(CN)₂, z. B. in Gegenwart von Pyridin in einem inerten Lösungsmittel wie z. B. DMF oder N-Methylpyrrolidon bei Temperaturen zwischen 20° und 200°.

Verbindungen der Formel I, worin A¹ durch mindestens ein F-Atom und/oder eine CN-Gruppe substituiert ist, können auch aus den entsprechenden Diazoniumsalzen durch Austausch der Diazoniumgruppe gegen ein Fluoratom oder gegen eine CN-Gruppe, z.B. nach den Methoden von Schiemann oder Sandmeyer, erhalten werden.

Dioxanderivate bzw. Dithianderivate der Formel I werden zwecksmäßig durch Reaktion eines entsprechenden Aldehyds (oder eines seiner reaktionsfähigen Derivate) mit einem entsprechenden 1,3-Diol (oder einem seiner reaktionsfähigen Derivate) bzw. einem entsprechenden 1,3-Dithiol hergestellt, vorzugsweise in Gegenwart eines inerten Lösungsmittels wie z.B. Benzol oder Toluol und/oder eines Katalysators, z.B. einer starken Säure wie Schwefelsäure, Benzol- oder p-Toluolsulfonsäure, bei Temperaturen zwischen etwa 20° und etwa 150°, vorzugsweise zwischen 80° und 120°. Als reaktionsfähige Derivate der Ausgangsstoffe eignen sich in erster Linie Acetale.

Die genannten Aldehyde und 1,3-Diole bzw. 1,3-Dithiole sowie ihre reaktionsfähigen Derivate sind zum Teil bekannt, zum Teil können sie ohne Schwierigkeiten nach Standardverfahren der Organischen Chemie aus literaturbekannten Verbindungen hergestellt werden. Beispielsweise sind die Aldehyde durch Oxydation entsprechender Alkohole oder durch Reduktion von Nitrilen oder entsprechenden Carbonsäuren oder ihrer Derivate, die Diole durch Reduktion entsprechender Diester und die Dithiole durch Umsetzung entsprechender Dihalogenide mit NaSH erhältlich.

Die Ausgangsmaterialien sind entweder bekannt oder können in Analogie zu bekannten Verbindungen hergestellt werden.

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexan-carbonsäurephenyl- oder cyclohexyl-ester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexene, Cyclohexylcyclohexylcyclohexene, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, l-Cyclohexyl-2-(4-phenyl-cyclohexyl)-ethane, 1-Cyclohexyl-2-biphenylylethane, 1-Phenyl-2-cyclohexylphenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4 und 5 charakterisieren:

R'-L-E-R" 1

R'-L-COO-E-R" 2

R'-L-OOC-E-R" 3

R'-L-CH₂CH₂-E-R" 4

R'-L-C=C-E-R" 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Rest L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe . -Phe-Cyc-, -Cyc-Cyc-, -G-Phe- und -G-Cyc-.

R' und R" bedeuten in einer kleineren Untergruppe der Verbindungen der Formeln 1, 2, 3, 4 und 5 jeweils unabhängig voneinander Alkyl, Alkenyl, Alkoxy, Alkoxyalkyl, Alkenyloxy oder Alkanoyloxy mit bis zu 8 Kohlenstoffatomen. Im folgenden wird diese kleinere Untergruppe Gruppe A genannt und die Verbindungen werden mit den Teilformeln la, 2a, 3a, 4a und 5a bezeichnet. Bei den meisten dieser Verbindungen sind R' und R" voneinander verschieden, wobei einer dieser Reste meist Alkyl, Alkenyl, Alkoxy oder Alkoxyalkyl ist.

In einer anderen als Gruppe B bezeichneten kleineren Untergruppe der Verbindungen der Formeln 1, 2, 3, 4 und 5 bedeutet R" -F, -Cl, -NCS oder -(O)ᵢCH₃₋₍ₖ₊₁₎FₖCl₁, wobei i 0 oder 1 und k+1 1, 2 oder 3 sind; die Verbindungen, in denen R" diese Bedeutung hat, werden mit den Teilformeln 1b, 2b, 3b, 4b und 5b bezeichnet. Besonders bevorzugt sind solche Verbindungen der Teilformeln 1b, 2b, 3b, 4b und 5b, in denen R" die Bedeutung -F, -Cl, -NCS, -CF₃, -OCHF₂ oder -OCF₃ hat.

In den Verbindungen der Teilformeln 1b, 2b, 3b, 4b und 5b hat R' die bei den Verbindungen der Teilformeln la-5a angegebene Bedeutung und ist vorzugsweise Alkyl, Alkenyl, Alkoxy oder Alkoxyalkyl.

In einer weiteren kleineren Untergruppe der Verbindungen der Formeln 1, 2, 3, 4 und 5 bedeutet R" -CN; diese Untergruppe wird im folgenden als Gruppe C bezeichnet und die Verbindungen dieser Untergruppe werden entsprechend mit Teilformeln lc, 2c, 3c, 4c und 5c beschrieben. In den Verbindungen der Teilformeln lc, 2c, 3c, 4c und 5c hat R' die bei den Verbindungen der Teilformeln la-5a angegebene Bedeutung und ist vorzugsweise Alkyl, Alkoxy oder Alkenyl.

Neben den bevorzugten Verbindungen der Gruppen A, B und C sind auch andere Verbindungen der Formeln 1, 2, 3, 4 und 5 mit anderen Varianten der vorgesehenen Substituenten gebräuchlich. All diese Substanzen sind nach literaturbekannten Methoden oder in Analogie dazu erhältlich.

Die erfindungsgemäßen Medien enthalten neben erfindungsgemäßen Verbindungen der Formel I vorzugsweise eine oder mehrere Verbindungen, welche ausgewählt werden aus der Gruppe A und/oder Gruppe B und/oder Gruppe C. Die Massenanteile der Verbindungen aus diesen Gruppen an den erfindungsgemäßen Medien sind vorzugsweise
- Gruppe A:: 0 bis 90 %, vorzugsweise 20 bis 90 %, insbesondere 30 bis 90 %
- Gruppe B:: 0 bis 80 %, vorzugsweise 10 bis 80 %, insbesondere 10 bis 65 %
- Gruppe C:: 0 bis 80 %, vorzugsweise 5 bis 80 %, insbesondere 5 bis 50 %
wobei die Summe der Massenanteile der in den jeweiligen erfindungsgemäßen Medien enthaltenen Verbindungen aus den Gruppen A und/oder B und/oder C vorzugsweise 5 %-90 % und insbesondere 10 % bis 90 % beträgt.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungsgemäßen Verbindungen. Die Medien enthalten vorzugsweise drei, vier oder fünf erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die folgenden Beispiele sollen die Erfindung erläutern, ohne sie zu begrenzen. mp. = Schmelzpunkt, cp. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Es bedeuten ferner:

K: Kristallin-fester Zustand, S: smektische Phase (der Index kennzeichnet den Phasentyp), N: nematischer Zustand, Ch: cholesterische Phase, I: isotrope Phase. Die zwischen zwei Symbolen stehende Zahl gibt die Umwandlungstemperatur in Grad Celsius an.
- DAST: Diethylaminoschwefeltrifluorid
- DCC: Dicyclohexylcarbodiimid
- DDQ: Dichlordicyanobenzochinon
- DIBALH: Diisobutylaluminiumhydrid
- KOT: Kalium-tertiär-butanolat
- THF: Tetrahydrofuran
- pTSOH: p-Toluolsulfonsäure
- NDBDH: 1,3-Dibrom-5,5-dimethylhydantoin

### Beispiel 1

1,1-Difluorethyl-4trans-4-(trans-4-propylcyclohexyl)cyclohexyl-phenyl-keton

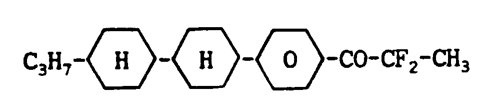

Zu 18,18 g 1-Brom-4-[trans-4(trans-4-propylcyclohexyl)-cyclohexyl]benzol in 300 ml Diethylether/THF 1:1 werden bei -70° 36 ml einer 15 %igen Lösung Butyllithium in Hexan zugetropft. Nach 1 Stunde Rühren werden (weiterhin bei -70°) 8,0 g l,l-Difluorpropionsäure-ethylester zugetropft. Nach weiteren 2 Stunden Rühren wird (immer noch bei -70°) eine Mischung auf 5 ml Ethanol und 5 ml 37 %iger Salzsäure zugetropft; das gesamte Gemisch wird dann in eine Mischung aus 200 ml 2 %iger Salzsäure und 100 ml Wasser eingegossen. Die organische Phase wird abgetrennt und im Vakuum eingedampft. Der Rückstand wurde nach Säulenchromatografie über Kieselgel aus Hexan umkristallisiert, K 92 N 197,1 I.

### Analog werden hergestellt:

1,1-Difluorethyl-4-[trans-4-(trans-4-ethylcyclohexyl)-cyclohexyl]-phenyl-keton
1,1-Difluorethyl-4-[trans-4-(trans-4-butylcyclohexyl)-cyclohexyl]-phenyl-keton
1,1-Difluorethyl-4-[trans-4-(trans-4-pentylcyclohexyl)-cyclohexyl]-phenyl-keton
1,1-Difluorethyl-4-[trans-4-(trans-4-hexylcyclohexyl)-cyclohexyl]-phenyl-keton
1,1-Difluorethyl-4-[trans-4-(trans-4-heptylcyclohexyl)-cyclohexyl]-phenyl-keton
1,1-Difluorethyl-4-[trans-4-(trans-4-octylcyclohexyl)-cyclohexyl]-phenyl-keton
1,1-Difluorethyl-4-[trans-4-(trans-4-nonylcyclohexyl)-cyclohexyl]-phenyl-keton
1,1-Difluorethyl-4-[trans-4-(trans-4-oct-3-enylcyclohexyl)-cyclohexyl]-phenyl-keton

### Beispiel 2

1,1-Difluorethyl-4-(trans-4-heptylcyclohexyl)-phenyl-keton

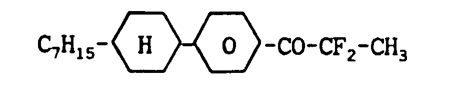

Herstellung analog Beispiel 1 unter Verwendung von 16,87 g 1-Brom-4-(trans-4-heptylcyclohexyl)benzol, 40,3 ml 15 %iger Butyllithium-Lösung in Hexan und 9,0 g 1,1-Difluorpropionsäure-ethylester, K 28 N 43,6 I.

### Analog werden hergestellt:

1,1-Difluorethyl-4-(trans-4-ethylcyclohexyl)-phenyl-keton
1,1-Difluorethyl-4 (trans-4-propylcyclohexyl)-phenyl-keton
1,1-Difluorethyl-4-(trans-4-butylcyclohexyl)-phenyl-keton
1,1-Difluorethyl-4-(trans-4-pentylcyclohexyl)-phenyl-keton
1,1-Difluorethyl-4-(trans-4-hexylcyclohexyl)-phenyl-keton
1,1-Difluorethyl-4-(trans-4-octylcyclohexyl)-phenyl-keton
1,1-Difluorethyl-4-(trans-4-nonylcyclohexyl)-phenyl-keton
1,1-Difluorethyl-4-(trans-4-alkylcyclohexyl)-phenyl-keton

### Beispiel 3

1,1-Difluorethyl-4-(trans-4-pentylcyclohexyl)-phenylphenylketon

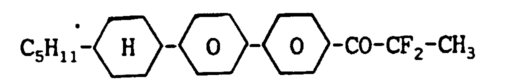

Herstellung aus 19,3 g 1-Brom-4-[4(trans-4-pentylcyclohexyl)phenyl]-benzol, sonst entsprechend wie bei Beispiel 2, K 52 S_{B} 140 S_{A} 169 N 190,7 I.

### Analog werden hergestellt:

1,1-Difluorethyl-4-[4-(trans-4-ethylcyclohexyl)-phenyl)-phenyl]-keton
1,1-Difluorethyl-4-[4-(trans-4-propylcyclohexyl)-phenyl)-phenyl]-keton
1,1-Difluorethyl-4-[4-(trans-4-butylcyclohexyl)-phenyl)-phenyl]-keton
1,1-Difluorethyl-4-[4-(trans-4-hexylcyclohexyl)-phenyl)-phenyl]-keton
1,1-Difluorethyl-4-[4-(trans-4-heptylcyclohexyl)-phenyl)-phenyl]-keton
1,1-Difluorethyl-4-[4-(trans-4-octylcyclohexyl)-phenyl)-phenyl]-keton
1,1-Difluorethyl-4-[4-(trans-4-nonylcyclohexyl)-phenyl)-phenyl]-keton
1,1-Difluorethyl-4-[4- (trans-4-but-3-enylcyclohexyl)-phenyl]-phenyl-keton

### Beispiel 4

1,1-Difluorbutyl-4-(trans-4-pentylcyclohexyl)-phenyl-keton

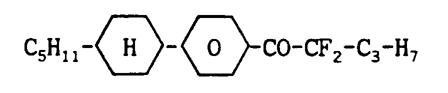

Herstellung analog Beispiel 1 unter Verwendung von 15,5 g 1-Brom-4-(trans-4-pentylcyclohexyl)benzol, 40,3 ml 15 %iger Butyllithium-Lösung in Hexan und 10,8 g 1,1-Difluor-valeriansäure-ethylester, K 20 S_{B} 33 N 53,9 I.
1,1-Difluorbutyl-4-(trans-4-ethylcyclohexyl)-phenyl-keton
1,1-Difluorbutyl-4-(trans-4-propylcyclohexyl)-phenyl-keton
1,1-Difluorbutyl-4-(trans-4-butylcyclohexyl)-phenyl-keton
1,1-Difluorbutyl-4-(trans-4-hexylcyclohexyl)-phenyl-keton
1,1-Difluorbutyl-4-(trans-4-heptylcyclohexyl)-phenyl-keton
1,1-Difluorbutyl-4-(trans-4-octylcyclohexyl)-phenyl-keton
1,1-Difluorbutyl-4-(trans-4-undecylcyclohexyl)-phenyl-keton

### Beispiel 5

1,1-Difluorbutyl-4-[trans-4(trans-4-propylcyclohexyl)-cyclohexyl]-phenyl-keton

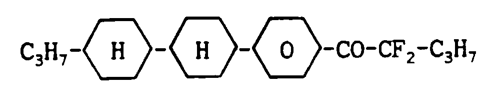

Herstellung analog Beispiel 1 unter Verwendung von 16,6 g 1-Brom-4-[trans-4(trans-4-propylcyclohexyl)-cyclohexyl]-benzol, 33 ml 15 %iger Butyllithium-Lösung und 8,86 g 1,1-Difluor-valeriansäure-ethylester, K 73 S_{B} 152 N 199 I.
1,1-Difluorbutyl-4-[trans-4-(trans-4-ethylcyclohexyl)-cyclohexyl]-phenyl-keton
1,1-Difluorbutyl-4-[trans-4-(trans-4-butylcyclohexyl)-cyclohexyl]-phenyl-keton
1,1-Difluorbutyl-4-[trans-4-(trans-4-pentylcyclohexyl)-cyclohexyl]-phenyl-keton
1,1-Difluorbutyl-4-[trans-4-(trans-4-hexylcyclohexyl)-cyclohexyl]-phenyl-keton
1,1-Difluorbutyl-4-[trans-4-(trans-4-heptylcyclohexyl)-cyclohexyl]-phenyl-keton
1,1-Difluorbutyl-4-[trans-4-(trans-4-octylcyclohexyl)-cyclohexyl]-phenyl-keton
1,1-Difluorbutyl-4-[trans-4-(trans-4-decylcyclohexyl)-cyclohexyl]-phenyl-keton

### Beispiel 6

1,1-Difluorbutyl-4[4(trans-4-pentylcyclohexyl)-phenyl-phenyl]-keton

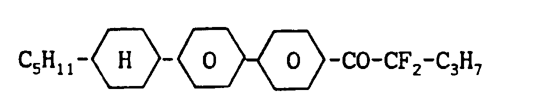

Herstellung analog Beispiel 1 unter Verwendung von 21,2 g 1-Brom-4-[4(trans-4-pentylcyclohexyl)-phenyl]-benzol, 43,7 ml 15 %iger Butyllithium-Lösung und 11,7 g 1,1-Difluor-valeriansäure-ethylester, K 159 S_{B} (158) S_{A} 189 N 192,8 I, Δε = +8,5 I
1,1-Difluorbutyl-4-[4-(trans-4-ethylcyclohexyl)-phenyl-phenyl]-keton
1,1-Difluorbutyl-4-[4-(trans-4-propylcyclohexyl)-phenyl-phenyl]-keton
1,1-Difluorbutyl-4-[4-(trans-4-butylcyclohexyl)-phenyl-phenyl]-keton
1,1-Difluorbutyl-4-[4-(trans-4-hexylcyclohexyl)-phenyl-phenyl]-keton
1,1-Difluorbutyl-4-[4-(trans-4-heptylcyclohexyl)-phenyl-phenyl]-keton
1,1-Difluorbutyl-4-[4-(trans-4-octylcyclohexyl)-phenyl-phenyl]-keton
l,l-Difluorbutyl-4-[4-(trans-4-nonylcyclohexyl)-phenyl-phenyl]-keton
1,1-Difluorbutyl-4-[4-(trans-4-hex-2-enylcyclohexyl)-phenyl-phenyl]-keton

### Beispiel 7

4(trans-4-Pentylcyclohexyl)-1-(1,2,2-trifluorpropyl)-benzol

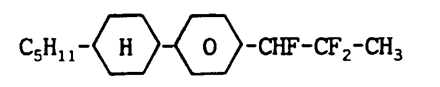

Zu einer Lösung von 14,8 g 1,1-Difluorethyl-4(trans-4-heptyl-cyclohexyl)phenyl-keton (Herstellung analog Beispiel 2) in 80 ml Isopropanol werden 0,7 g (18,5 mmol) Natriumboranat zugegeben. Nach 2 Stunden Rühren bei Raumtemperatur wird mit verdünnter Salzsäure angesäuert; nach Zugabe von 500 ml Wasser wird mit Ether extrahiert und die Etherextrakte im Vakuum eingedampft. 6,8 g des erhaltenen, rohen Zwischenproduktes, 5,0 ml DAST und 5 ml Dichlormethan werden 15 Stunden am Rückfluß gekocht. Anschließend wird vorsichtig Wasser zugetropft. Die organische Phase wird abgetrennt und im Vakuum eingedampft. Der Rückstand wird über eine Kieselgel-Säule mit Petrolether chromatografiert. Das Eluat wird im Vakuum eingedampft und der Rückstand im Vakuum (0,1 mbar) destilliert, K 13 I.

### Analog werden hergestellt:

4-(trans-4-Ethylcyclohexyl)-1-(1,2,2-trifluorpropyl)-benzol
4-(trans-4-Propylcyclohexyl)-1-(1,2,2-trifluorpropyl)-benzol
4-(trans-4-Butylcyclohexyl)-1-(1,2,2-trifluorpropyl)-benzol
4-(trans-4-Hexylcyclohexyl)-1-(1,2,2-trifluorpropyl)-benzol
4-(trans-4-Heptylcyclohexyl)-1-(1,2,2-trifluorpropyl)-benzol
4-(trans-4-Octylcyclohexyl)-1-(1,2,2-trifluorpropyl)-benzol
4-(trans-4-Allyloxycyclohexyl)-1-(1,2,2-trifluorpropyl)-benzol

### Beispiel 8

4-[4-(trans-4-Pentylcyclohexyl)-phenyl]-1(1,2,2-trifluorpropyl)benzol

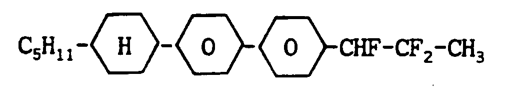

Zu einer Lösung von 7,2 g der in Beispiel 3 hergestellten Verbindung in 30 ml Isopropanol werden 0,3 g Natriumboranat gegeben. Nach 2 Stunden Rühren bei Raumtemperatur wird auf Wasser gegossen, mit Ether extrahiert und die Etherextrakte im Vakuum eingedampft. Nach Umkristallisieren aus Hexan erhält man 4,0 g Zwischenprodukt. Dieses wurde, wie bei Beispiel 7 beschrieben, weiter umgesetzt und aufgearbeitet; der Rückstand wird zweimal aus Ethanol umkristallisiert, K 147 I.

### Analog werden hergestellt.

4-[4-(trans-4-Ethylcyclohexyl)-phenyl]-1-(1,2,2-trifluorpropyl)-benzol
4-[4-(trans-4-Propylcyclohexyl)-phenyl]-propyl)-benzol
4-[4-(trans-4-Butylcyclohexyl)-phenyl]-1-(1,2,2-trifluorpropyl)-benzol
4-[4-(trans-4-Hexylcyclohexyl)-phenyl]-1-(1,2,2-trifluorpropyl)-benzol
4-[4-(trans-4-Heptylcyclohexyl)-phenyl]-1-(1,2,2-trifluorpropyl)-benzol
4-[4-(trans-4-Octylcyclohexyl)-phenyl]-1-(1,2,2-trifluorpropyl)-benzol
4-[4-(trans-4-Dodecylcyclohexyl)-phenyl]-1-(1,2,2-trifluorpropyl)-benzol
4-[4-(trans-4-Pentylcyclohexyl)-phenyl]-propyl)-benzol, K 137 N 137,6 I

### Beispiel 9

4(trans-4-Pentylcyclohexyl)-1-(1,2,2-trifluorpentyl)benzol.

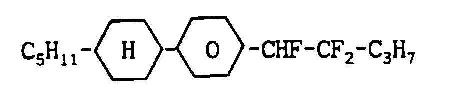

Ausgehend von 4,7 g der in Beispiel 4 hergestellten Verbindung und 0,25 g Natriumboranat, ferner 5,0 ml DAST Versuchsdurchführung wie bei Beispiel 7, K 40 I, Δε = +2,1.

### Analog werden hergestellt:

4-(trans-4-Ethylcyclohexyl)-1-(1,2,2-trifluorpentyl)-benzol
4-(trans-4-Propylcyclohexyl)-1-(1,2,2-trifluorpentyl)-benzol
4-(trans-4-Butylcyclohexyl)-1-(1,2,2-trifluorpentyl)-benzol
4-(trans-4-Hexylcyclohexyl)-1-(1,2,2-trifluorpentyl)-benzol
4-(trans-4-Heptylcyclohexyl)-1-(1,2,2-trifluorpentyl)-benzol
4-(trans-4-Octylcyclohexyl)-1-(1,2,2-trifluorpentyl)-benzol
4-(trans-4-Oct-3-enylcyclohexyl)-1-(1,2,2-trifluorpentyl)-benzol

### Beispiel 10

4[trans-4-(trans-4-Propylcyclohexyl)cyclohexyl]-1(1,2,2-trifluorpentyl)benzol

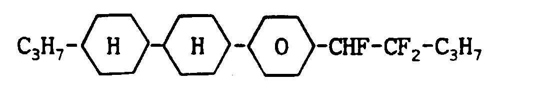

Ausgehend von 4,0 g der in Beispiel 5 hergestellen Verbindung und 0,15 g (4,0 mmol) Natriumboranat, ferner 5,0 ml (37,8 mmol) DAST Versuchsdurchführung wie bei Beispiel 8, K 56 S_{B} 166 I.

### Analog werden hergestellt:

4-[trans-4-(trans-4-Ethylcyclohexyl)-cyclohexyl]-1-(1,2,2-trifluorpentyl)-benzol
4-[trans-4-(trans-4-Butylcyclohexyl)-cyclohexyl]-1-(1,2,2-trifluorpentyl)-benzol
4-[trans-4-(trans-4-Pentylcyclohexyl)-cyclohexyl]-1-(1,2,2-trifluorpentyl)-benzol
4-[trans-4-(trans-4-Hexylcyclohexyl)-cyclohexyl]-1-(1,2,2-trifluorpentyl)-benzol
4-[trans-4-(trans-4-Heptylcyclohexyl)-cyclohexyl]-1-(1,2,2-trifluorpentyl)-benzol
4-[trans-4-(trans-4-Octylcyclohexyl)-cyclohexyl]-1-(1,2,2-trifluorpentyl)-benzol
4-[trans-4-(trans-4-Ethoxycyclohexyl)-cyclohexyl]-1-(1,2,2-trifluorpentyl)-benzol

### Beispiel 11

4[4-(trans-4-Pentylcyclohexyl)-phenyl]-1-(1,2,2-trifluorpentyl)-benzol

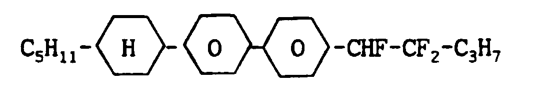

Ausgehend von 4,3 g der in Beispiel 6 hergestellen Verbindung und 0,19 g (5,0 mmol) Natriumboranat, ferner 5,0 ml (37,8 mmol) DAST Versuchsdurchführung wie bei Beispiel 8, K 148 I, Δε = +5,4.

### Analog werden hergestellt:

4-[4-(trans-4-Ethylcyclohexyl)-phenyl]-1-(1,2,2-trifluorpentyl)-benzol
4-[4-(trans-4-Propylcyclohexyl)-phenyl]-1-(1,2,2-trifluorpentyl)-benzol
4-[4-(trans-4-Butylcyclohexyl)-phenyl]-1-(1,2,2-trifluorpentyl)-benzol
4-[4-(trans-4-Hexylcyclohexyl)-phenyl]-1-(1,2,2-trifluorpentyl)-benzol
4-[4-(trans-4-Heptylcyclohexyl)-phenyl]-1(1,2,2-trifluorpentyl)-benzol
4-[4-(trans-4-Octylcyclohexyl)-phenyl]-1-(1,2,2-trifluorpentyl)-benzol

### Beispiel 12

4-(1,1-Difluorethyl)-4'-pentylbiphenyl

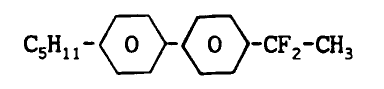

Zu einer Mischung aus 15,6 g (4-Acetyl-4'-pentyl-biphenyl und 9,0 ml Ethandithiol werden 7,3 ml Bortrifluorid-Diethylether-Addukt zugetropft. Nach 15 Stunden Rühren wird mit 200 ml Hexan verdünnt und dreimal mit 16 %iger Natronlauge, einmal mit gesättigter Kochsalz-Lösung sowie einmal mit Wasser gewaschen. Die organische Phase wird im Vakuum eingedampft, der Rückstand aus Ethanol umkristallisiert. Ausbeute am Zwischenprodukt: 13,1 g. Zu einer Lösung von 11,0 g 1,3-Dibrom-5,5-dimethylhydantoin in 100 ml Dichlormethan werden bei -70° 16,8 ml Fluorwasserstoff-Pyridin-Komplex (64 %ig in HF) zugetropft. Dann wird eine Lösung des Zwischenproduktes in 30 ml Dichlormethan zugetropft. Es wird noch 30 min bei -70° gerührt, dann auf Raumtemperatur erwärmt, dreimal mit Wasser gewaschen und schließlich die organische Phase im Vakuum eingedampft. Der Rückstand wird über eine Kieselgelsäule mit Hexan chromatografiert. Das Eluat wird im Vakuum eingedampft und der Rückstand zweimal aus Ethanol umkristallisiert.

### Analog werden hergestellt:

4-(1,1-Difluorethyl)-4'-ethylbiphenyl
4-(1,1-Difluorethyl)-4'-propylbiphenyl
4-(1,1-Difluorethyl)-4'-butylbiphenyl
4-(1,1-Difluorethyl)-4'-hexylbiphenyl
4-(1,1-Difluorethyl)-4'-heptylbiphenyl
4-(1,1-Difluorethyl)-4'-octylbiphenyl
4-(1,1-Difluorethyl)-4'-ethoxybiphenyl
4-(1,1-Difluorethyl)-4'-propyloxybiphenyl
4-(1,1-Difluorethyl)-4'-butyloxybiphenyl
4-(1,1-Difluorethyl)-4'-pentyloxybiphenyl
4-(1,1-Difluorethyl)-4'-hexyloxybiphenyl
4-(1,1-Difluorethyl)-4'-heptyloxybiphenyl
4-(1,1-Difluorethyl)-4'-octyloxybiphenyl
4-(1,1-Difluorethyl)-4'-oct-3-enylbiphenyl
4-(1,1-Difluorpropyl)-4'-pentylbiphenyl
4-(1,1-Difluorbutyl)-4'-pentylbiphenyl
4-(1,1-Difluorpentyl)-4'-pentylbiphenyl
4-(1,1-Difluorhexyl)-4'-pentylbiphenyl
4-(1,1-Difluorheptyl)-4'-pentylbiphenyl
4-(1,1-Difluoroctyl)-4'-pentylbiphenyl

### Beispiel 13

4-(trans,trans-4'-Propylbicyclohexyl-4-yl)-1-(1,1-difluorethyl)-2,6-difluorbenzol

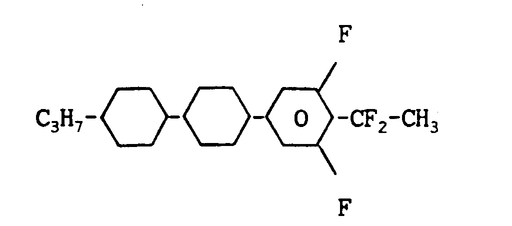

### 13A 3-(trans,trans-4'-Propylbicyclohexyl(4-yl)-1,5-difluorbenzol

Zu einer Suspension von 6,0 g Magnesiumspänen in 60 ml Ether gibt man 2 Tropfen Brom. Anschließend wird eine Lösung von 48,2 g 3,5-Difluorbrombenzol in 60 ml Ether zugetropft. Man rührt 0,5 h nach und tropft dann bei 20-25 °C eine Lösung von 44,5 g 4-trans-(4-Propylcyclohexyl)-cyclohexanon in 50 ml Ether zum Grignardreagenz. Es wird 2 Stunden nachgerührt, auf 500 ml Wasser gegossen, mit 30 ml konz. Salzsäure angesäuert und mitEther ausgeschüttelt. Die organische Phase wird zum Rückstand eingedampft und anschließend mit 1000 ml Toluol und 120 ml 20%iger Schwefelsäure 1 h am Rückfluß gekocht.

Nach Phasentrennung und Neutralisation mit ges. NaHCO₃- Lösung wird mit 10 g Pt/C 5 % bei 1 bar und 60 °C hydriert.

Anschließend wird filtriert und eingeengt. Nach flash Chromatographie erhält man das reine Produkt 13A.

### 13B 4-(trans,trans-4'-Propylbicyclohexyl-4'-yl)-2,6-difluoracetophenon

Eine Lösung von 35,3 g in 350 ml Ether wird unter Stickstoff auf -70 °C gekühlt und 69 ml einer 1,6 n Lösung von BuLi in Hexan werden bei dieser Temperatur zugetropft. Es wird 0,5 h nachgerührt. Dann werden 34,0 g (110 mmol) Mangan(II)-jodid zum Reaktionsgemisch gegeben, und es wird 60 min bei -70 °C nachgerührt. Man läßt auf -50 °C erwärmen und tropft dann eine Lösung von 8,6 g (110 mmol) Acetylchlorid in 27 ml Ether zu. Man läßt unter Rühren auf Raumtemperatur erwärmen und rührt noch 2 h nach. Es wird mit verd. Schwefelsäure hydrolysiert. Die organische Phase wird abgetrennt, getrocknet (Na₂SO₄) und zum Rückstand eingeengt. Nach Umkristallisation erhält man das reine Acetophenon 13B.

### 13C 2[4-(trans,trans-4'-Propylbicyclohexyl-4-yl)-2,6-difluorphenyl]-2-methyl-1,3-dithiolon

In eine Lösung von 30,3 g 13B und 8,5 g 1,2-Ethandithiol in 200 ml Dichlormethan wird über 6 h langsam Chlorwasserstoff eingeleitet. Dann wird dreimal mit je 200 ml Wasser und einmal mit ges. Natriumhydrogencarbonatlösung gewaschen. Nach Trocknen (Na₂SO₄), Einengen und Umkristallisation erhält man 13C.

### 13D

Unter Stickstoff werden 5,7 g NDBDH in 40 ml Dichlormethan gelöst. Man kühlt auf -78 °C und gibt nacheinander 12,3 ml einer 65%igen Lösung von Fluorwasserstoff in Pyridin und tropfenweise eine Lösung von 9,0 g 13C in 45 ml Dichlormethan zu. Nach 0,5 h verdünnt man mit Hexan und filtert über basisches Aluminiumoxid. Nach flash Chromatographie erhält man das reine Produkt.

### Analog werden hergestellt:

4-(trans,trans-4'-Ethylbicyclohexyl-4-yl)-1-(1,1-difluorethyl)-2,6-difluorbenzol
4-(trans,trans-4'-Butylbicyclohexyl-4-yl)-1-(1,1-difluorethyl)-2,6-difluorbenzol
4-(trans,trans-4'-Pentylbicyclohexyl-4-yl)-1-(1,1-difluorethyl)-2,6-difluorbenzol
4-(trans,trans-4'-Hexylbicyclohexyl-4-yl)-1-(1,1-difluorethyl)-2,6-difluorbenzol
4-(trans,trans-4'-Heptylbicyclohexyl-4-yl)-1-(1,1-difluorethyl)-2,6-difluorbenzol
4-(trans,trans-4'-Octylbicyclohexyl-4-yl)-1-(1,1-difluorethyl)-2,6-difluorbenzol
4-(trans,trans-4'-Ethylbicyclohexyl-4-yl)-1-(1,1-difluorethyl)-2-difluorbenzol
4-(trans,trans-4'-Butylbicyclohexyl-4-yl)-1-(1,1-difluorethyl)-2-difluorbenzol
4-(trans,trans-4'-Pentylbicyclohexyl-4-yl)-1-(1,1-difluorethyl)-2-difluorbenzol
4-(trans,trans-4'-Hexylbicyclohexyl-4-yl)-1-(1,1-difluorethyl)-2-difluorbenzol
4-(trans,trans-4'-Heptylbicyclohexyl-4-yl)-1-(1,1-difluorethyl)-2-difluorbenzol
4-(trans,trans-4'-Octylbicyclohexyl-4-yl)-1-(1,1-difluorethyl)-2-difluorbenzol
4-(trans,trans-4'-Ethylbicyclohexyl-4-yl)-1-(1,1-difluorpentyl)-2-difluorbenzol

4-(trans,trans-4'-Butylbicyclohexyl-4-yl)-1-(1,1-difluorpentyl)-2-difluorbenzol
4-(trans,trans-4'-Pentylbicyclohexyl-4-yl)-1-(1,1-difluorpentyl)-2-difluorbenzol
4-(trans,trans-4'-Hexylbicyclohexyl-4-yl)-1-(1,1-difluorpentyl)-2-difluorbenzol
4-(trans,trans-4'-Heptylbicyclohexyl-4-yl)-1-(1,1-difluorpentyl)-2-difluorbenzol
4-(trans,trans-4'-Octylbicyclohexyl-4-yl)-1-(1,1-difluorpentyl)-2-difluorbenzol

4-(trans,trans-4'-Ethylbicyclohexyl-4-yl)-1-(1,1-difluorpentyl)-2,6-difluorbenzol
4-(trans,trans-4'-Butylbicyclohexyl-4-yl)-1-(1,1-difluorpentyl)-2,6-difluorbenzol
4-(trans,trans-4'-Pentylbicyclohexyl-4-yl)-1-(1,1-difluorpentyl)-2,6-difluorbenzol
4-(trans,trans-4'-Hexylbicyclohexyl-4-yl)-1-(1,1-difluorpentyl)-2,6-difluorbenzol
4-(trans,trans-4'-Heptylbicyclohexyl-4-yl)-1-(1,1-difluorpentyl)-2,6-difluorbenzol
4-(trans,trans-4'-Octylbicyclohexyl-4-yl)-1-(1,1-difluorpentyl)-2,6-difluorbenzol

4'-(4-trans-Propylcyclohexyl)-4-(1,1-difluorethyl)-3-fluorbiphenyl
4'-(4-trans-Ethylcyclohexyl)-4-(1,1-difluorethyl)-3-fluorbiphenyl
4'-(4-trans-Butylcyclohexyl)-4-(1,1-difluorethyl)-3-fluorbiphenyl
4'-(4-trans-Pentylcyclohexyl)-4-(1,1-difluorethyl)-3-fluorbiphenyl
4'-(4-trans-Hexylcyclohexyl)-4-(1,1-difluorethyl)-3-fluorbiphenyl
4'-(4-trans-Heptylcyclohexyl)-4-(1,1-difluorethyl)-3-fluorbiphenyl
4'-(4-trans-Octylcyclohexyl)-4-(1,1-difluorethyl)-3-fluorbiphenyl

4'-(4-trans-Ethylcyclohexyl)-4-(1,1-difluorpentyl)-3-fluorbiphenyl
4'-(4-trans-Propylcyclohexyl)-4-(1,1-difluorpentyl)-3-fluorbiphenyl
4'-(4-trans-Butylcyclohexyl)-4-(1,1-difluorpentyl)-3-fluorbiphenyl
4'-(4-trans-Pentylcyclohexyl)-4-(1,1-difluorpentyl)-3-fluorbiphenyl
4'-(4-trans-Hexylcyclohexyl)-4-(1,1-difluorpentyl)-3-fluorbiphenyl
4'-(4-trans-Heptylcyclohexyl)-4-(1,1-difluorpentyl)-3-fluorbiphenyl
4'-(4-trans-Octylcyclohexyl)-4-(1,1-difluorpentyl)-3-fluorbiphenyl

4'-(4-trans-Ethylcyclohexyl)-4-(1,1-difluorpentyl)-2'-fluorbiphenyl
4'-(4-trans-Propylcyclohexyl)-4-(1,1-difluorpentyl)-2'-fluorbiphenyl
4'-(4-trans-Butylcyclohexyl)-4-(1,1-difluorpentyl)-2'-fluorbiphenyl
4'-(4-trans-Pentylcyclohexyl)-4-(1,1-difluorpentyl)-2'-fluorbiphenyl
4'-(4-trans-Hexylcyclohexyl)-4-(1,1-difluorpentyl)-2'-fluorbiphenyl
4'-(4-trans-Heptylcyclohexyl)-4-(1,1-difluorpentyl)-2'-fluorbiphenyl
4'-(4-trans-Octylcyclohexyl)-4-(1,1-difluorpentyl)-2'-fluorbiphenyl

### Beispiel 14

### 4-(trans-4-Pentylcyclohexyl)-1-(1,1-difluor-2-oxo-propyl)-benzol

Zu einer Suspension von 0,15 mol Aluminiumchlorid in 500 ml Dichormethan werden unter heftigem Rühren nacheinander 0,1 mol trans-4-Pentylcyclohexylbenzol und 0,1 mol Oxalsäuremonoethylestermonochlorid bei 40 °C gegeben.

Nach Ansäuern mit verdünnter Salzsäure erhält man 2-[4-(trans-4-Pentylcyclohexyl)-phenyl]-2-oxoessigsäureethylester.

8 g des erhaltenen Zwischenproduktes werden mit 5,0 ml DAST und 45 ml Dichlormethan 15 Stunden auf 40 °C erhitzt. Nach üblicher Aufarbeitung erhält man 2-[4-(trans-4-Pentylcyclohexyl)-phenyl]-2,2-difluoressigsäureethylester. 10 mol dieses Produkts werden bei -78 °C zu einer Lösung von Methylmagnesiumjodid (hergestellt aus 10 mmol Mg und 10 mmol Jodmethan) in 20 ml THF gegeben und 4 h gerührt. Nach Aufwärmen auf Raumtemperatur und üblicher Aufarbeitung erhält man das Produkt:

### Analog werden hergestellt:

4-(trans-Pentylcyclohexyl)-1-(1,1-difluor-2-oxopentyl)
4-(trans-Heptylcyclohexyl)-1-(1,1-difluor-2-oxobenzol)
4'-Heptyl-4-(1,1-difluor-2-oxopropyl)-biphenyl
4'-Heptyl-4-(1,1-difluor-2-oxopentyl)-biphenyl
4-(trans,trans-4'-Pentylbicyclohexyl-4-yl)-1-(1,1-difluor-2-oxopropyl)-benzol
4-(trans,trans-4'-Propy1bicyc1ohexy1-4-y1)-1-(1,1-difluor-2-oxopentyl)-benzol

### Beispiel 15

### 4-(trans,trans-4'-Propylbicyclohexyl-4-yl)-1-(1,1,2,2-tetrafluorpropyl)-benzol

### Schritt 1

Zu 18,18 g 1-Brom-4-[trans-4-(trans-4-propylcyclohexyl)-cyclohexyl]-benzol in 300 ml Diethylether/THF 1:1 werden bei -70° 36 ml einer 15%igen Lösung Butyllithium in Hexan zugetropft. Nach 1 Stunde Rühren werden 8,0 g 1,1-Difluorpropionsäure-ethylester zugetropft. Nach weiteren 2 Stunden Rühren wird eine Mischung aus 5 ml Ethanol und 5 ml 37%iger Salzsäure zugetropft; das gesamte Gemisch wird dann in eine Mischung aus 200 ml 2%iger Salzsäure und 100 ml Wasser eingegossen. Die organische Phase wurde abgetrennt und im Vakuum eingedampft. Der Rückstand wird nach Säulenchromatographie über Kieselgel (mit Petrolether aus Eluens) aus Hexan umkristallisiert.

### Schritt 2 .

Eine Mischung aus 4,2 g der nach Schritt 1 erhaltenen Verbindung 1,5 ml DAST und 10 ml Hexan wird ca. 15 Stunden unter Rückfluß gekocht. Dann wird auf Wasser gegossen, mit Ether extrahiert, die Etherextrakte im Vakuum eingedampft und der Rückstand über eine Kieselgelsäule mit Petrolether chromatographiert. Das Eluat wurde im Vakuum eingedampft und der Rückstand aus Ethanol umkristallisiert, K 162 I Δε = +5,3.

### Analog werden hergestellt:

4-(trans,trans-4'-Ethylbicyclohexyl-4-yl)-1-(1,1,2,2-tetrafluorpropyl)-benzol

4-(trans,trans-4'-Butylbicyclohexyl-4-yl)-1-(1,1,2,2-tetrafluorpropyl)-benzol
4-(trans,trans-4'-Pentylbicyclohexyl-4-yl)-1-(1,1,2,2-tetrafluorpropyl)-benzol
4-(trans,trans-4'-Hexylbicyclohexyl-4-yl)-1-(1,1,2,2-tetrafluorpropyl)-benzol
4-(trans,trans-4'-Heptylbicyclohexyl-4-yl)-1-(1,1,2,2-tetrafluorpropyl)-benzol
4-(trans,trans-4'-Octylbicyclohexyl-4-yl)-1-(1,1,2,2-tetrafluorpropyl)-benzol
4-(trans,trans-4'-Ethylbicyclohexyl-4-yl)-1-(1,1,2,2-tetrafluorpentyl)-benzol, K 49 S_{B} 168 I, Δε = +5,1
4-(trans,trans-4'-Butylbicyclohexyl-4-yl)-1-(1,1,2,2-tetrafluorpentyl)-benzol
4-(trans,trans-4'-Pentylbicyclohexyl-4-yl)-1-(1,1,2,2-tetrafluorpentyl)-benzol
4-(trans,trans-4'-Hexylbicyclohexyl-4-yl)-1-(1,1,2,2-tetrafluorpentyl)-benzol
4-(trans,trans-4'-Heptylbicyclohexyl-4-yl)-1-(1,1,2,2-tetrafluorpentyl)-benzol
4-(trans,trans-4'-Octylbicyclohexyl-4-yl)-1-(1,1,2,2-tetrafluorpentyl)-benzol

### Beispiel 16

### 4-(trans-4-Heptylcyclohexyl)-1-(1,1,2,2-tetrafluorpropyl)-benzol

### Schritt 1

Herstellung analog Beispiel 15, Schritt 1, unter Verwendung von 16,87 g 1-Brom-4-(trans-4-heptylcyclohexyl)-benzol, 40,3 ml 15%iger Butyllithium-Lösung in Hexan und 9,0 g 1,1-Difluorpropionsäure-ethylester.

### Schritt 2

Eine Mischung aus 9,0 g dieser Verbindung 3,4 ml DAST und 10 ml Hexan wird 2 Stunden bei 50° gerührt. Dann wird auf Wasser gegossen, mit Ether extrahiert. Die Etherextrakte werden im Vakuum eingedampft, der Rückstand im Vakuum destilliert, K 1 I, Δε = +1,8.

### Analog werden hergestellt:

4-(trans-4-Ethylcyclohexyl)-1-(1,1,2,2-tetrafluorpropyl)-benzol
4-(trans-4-Propylcyclohexyl)-1-(1,1,2,2-tetrafluorpropyl)-benzol
4-(trans-4-Butylcyclohexyl)-1-(1,1,2,2-tetrafluorpropyl)-benzol
4-(trans-4-Pentylcyclohexyl)-1-(1,1,2,2-tetrafluorpropyl)-benzol
4-(trans-4-Hexylcyclohexyl)-1-(1,1,2,2-tetrafluorpropyl)-benzol
4-(trans-4-Octylcyclohexyl)-1-(1,1,2,2-tetrafluorpropyl)-benzol
4-(trans-4-Ethylcyclohexyl)-1-(1,1,2,2-tetrafluorpentyl)-benzol
4-(trans-4-Propylcyclohexyl)-1-(1,1,2,2-tetrafluorpentyl)-benzol
4-(trans-4-Butylcyclohexyl)-1-(1,1,2,2-tetrafluorpentyl)-benzol
4-(trans-4-Pentylcyclohexyl)-1-(1,1,2,2-tetrafluorpentyl)-benzol, K 16 I, Δε = +2,9
4-(trans-4-Hexylcyclohexyl)-l-(1,1,2,2-tetrafluorpentyl)-benzol
4-(trans-4-Octylcyclohexyl)-1-(1,1,2,2-tetrafluorpentyl)-benzol

### Beispiel 17

### 4'-(trans-4-Pentylcyclohexyl)-4-(1,1,2,2-tetrafluorpropyl)-biphenyl

### Schritt 1

Herstellung aus 19,3 g 1-Brom-4-4(trans-4-4-pentylcyclohexyl)-biphenyl-benzol, sonst entsprechend wie bei Beispiel 15, Schritt 1.

### Schritt 2

Ausgehend von 6,3 g dieser Verbindung und 2,1 ml DAST Versuchsdurchführung wie bei Beispiel 15, Schritt 2, K 147 I, Δε = +5,8.

### Analog werden hergestellt:

4'-(trans-4-Ethylcyclohexyl)-4-(1,1,2,2-tetrafluorpropyl)-biphenyl
4'-(trans-4-Propylcyclohexyl)-4-(1,1,2,2-tetrafluorpropyl)-biphenyl
4'-(trans-4-Butylcyclohexyl)-4-(1,1,2,2-tetrafluorpropyl)-biphenyl
4'-(trans-4-Hexylcyclohexyl)-4-(1,1,2,2-tetrafluorpropyl)-biphenyl
4'-(trans-4-Heptylcyclohexyl)-4-(1,1,2,2-tetrafluorpropyl)-biphenyl
4'-(trans-4-Octylcyclohexyl)-4-(1,1,2,2-tetrafluorpropyl)-biphenyl

4'-(trans-4-Ethylcyclohexyl)-4-(1,1,2,2-tetrafluorpentyl)-biphenyl
4'-(trans-4-Propylcyclohexyl)-4-(1,1,2,2-tetrafluorpentyl)-biphenyl
4'-(trans-4-Butylcyclohexyl)-4-(1,1,2,2-tetrafluorpentyl)-biphenyl
4'-(trans-4-Pentylcyclohexyl)-4-(1,1,2,2-tetrafluorpentyl)-biphenyl 4'-(trans-4-Hexylcyclohexyl)-4-(1,1,2,2-tetrafluorpentyl)-biphenyl
4'-(trans-4-Heptylcyclohexyl)-4-(1,1,2,2-tetrafluorpentyl)-biphenyl
4'-(trans-4-Octylcyclohexyl)-4-(1,1,2,2-tetrafluorpentyl)-biphenyl

4'-(trans-4-Ethylcyclohexyl)-4-(1,1,2,2-tetrafluorethyl)-biphenyl
4'-(trans-4-Propylcyclohexyl)-4-(1,1,2,2-tetrafluorethyl)-biphenyl
4'-(trans-4-Butylcyclohexyl)-4-(1,1,2,2-tetrafluorethyl)-biphenyl
4'-(trans-4-Butylcyclohexyl)-4-(1,1,2,2-tetrafluorethyl)-biphenyl
4'-(trans-4-Pentylcyclohexyl)-4-(1,1,2,2-tetrafluorethyl)-biphenyl, K 114 I, Δε = +10,0
4,-(trans-4-Heptylcyclohexyl)-4-(1,1,2,2-tetrafluorethyl)-biphenyl
4'-(trans-4-Octylcyclohexyl)-4-(1,1,2,2-tetrafluorethyl)-biphenyl

### Reference 2 - Beispiel 1

### Schritt 1

Zu einer Lösung von 30,9 g 1-Brom-4-(trans-4-pentylcyclohexyl)-benzol in 500 ml THF/Diethylether 1:1 werden bei -70° 80 ml einer 15%igen Butyllithium-Lösung in Hexan zugetropft. Nach 1 Stunde Rühren werden weiterhin bei -70° 9,1 ml Propenoxid zugetropft. Nach Erwärmen auf Raumtemperatur wird auf Wasser gegossen, mit verd. Salzsäure angesäuert, mit Ether extrahiert und die Etherextrakte im Vakuum eingedampft. Der Rückstand wird über eine Kieselgelsäure mit Petrolether/Essigester 9:1 chromatographiert. Das Eluat wird aus Hexan umkristallisiert.

### Beispiel 18

### 4'-(trans-4-Pentylcyclohexyl)-4-(2,2-difluorpropyl)-biphenyl

5,0 g des in Reference Beispiel 1 erhaltenen Zwischenproduktes werden mit PCC oxidiert.

Eine Mischung des so erhaltenen Ketons, 1,5 ml DAST und 10 ml Hexan wird 15 Stunden unter Rückfluß gekocht. Dann wird auf Wasser gegossen, mit Ether extrahiert, die Etherextrakte im Vakuum eingedampft und der Rückstand über eine Kieselgelsäule chromatographiert. Das Eluat wird im Vakuum eingedampft und der Rückstand aus Ethanol umkristallisiert, K 148 I, Δε = +6,9.

### Analog werden hergestellt:

4'-(trans-4-Ethylcyclohexyl)-4-(2,2-difluorpropyl)-biphenyl
4'-(trans-4-Propylcyclohexyl)-4-(2,2-difluorpropyl)-biphenyl
4'-(trans-4-Butylcyclohexyl)-4-(2,2-difluorpropyl)-biphenyl
4'-(trans-4-Hexylcyclohexyl)-4-(2,2-difluorpropyl)-biphenyl
4'-(trans-4-Heptylcyclohexyl)-4-(2,2-difluorpropyl)-biphenyl
4'-(trans-4-Ocytlcyclohexyl)-4-(2,2-difluorpropyl)-biphenyl
4'-(trans-4-Ethylcyclohexyl)-4-(2,2-difluoroctyl)-biphenyl
4'-(trans-4-Propylcyclohexyl)-4-(2,2-difluoroctyl)-biphenyl
4'-(trans-4-Butylcyclohexyl)-4-(2,2-difluoroctyl)-biphenyl
4'-(trans-4-Hexylcyclohexyl)-4-(2,2-difluoroctyl)-biphenyl
4'-(trans-4-Heptylcyclohexyl)-4-(2,2-difluoroctyl)-biphenyl
4'-(trans-4-Ocytlcyclohexyl)-4-(2,2-difluoroctyl)-biphenyl.

## Patentansprüche

1. Partiell fluorierte Verbindungen der Formel I,
R¹-A¹-Z¹-(A²-Z²)ₘ-A³-(Y)ₙ-Q-R²
wobei
A¹, A² und A³ jeweils unabhängig voneinander einen
(a) trans-1,4-Cyclohexylenrest, worin auch eine oder mehrere nicht benachbarte CH₂- Gruppen durch -O- und/oder -S- ersetzt sein können,
(b) 1,4-Phenylenrest, worin auch eine oder zwei CH-Gruppen durch N ersetzt sein können,
(c) Rest aus der Gruppe 1,3-Cyclobutylen, 1,3-Bicyclo(1,1,1)pentylen, 1,4-Cyclohexenylen, 1,4-Bicyclo(2,2,2)octylen, Piperidin-1,4-diyl, Naphthalin-2,6-diyl, Decahydronaphthalin-2,6-diyl und 1,2, 3,4-Tetrahydronaphthalin-2,6-diyl,
wobei die Reste (a) und (b) durch CN oder Halogen substituiert sein können,
R¹ und R² jeweils unabhängig voneinander einen unsubstituierten oder einen einfach durch CN, Halogen oder CF₃ substituierten Alkyl- oder Alkenylrest mit bis zu 15 C-Atomen, wobei in diesen Resten auch eine oder mehrere CH₂-Gruppen jeweils unabhängig voneinander durch -S-, -O-, -CO-, -CO-O-, -O-CO- oder -O-CO-O- so ersetzt sein können, daß S- und/oder O-Atome nicht direkt miteinander verknüpft sind, einer der Reste R¹ und R² auch H,
Z¹ und Z² jeweils unabhängig voneinander -CH₂CH₂-, -C≡C-, -CH₂O-, -OCH₂-, -CO-O-, -O-CO-, -CH=N-, -N=CH-, -CH₂S-, -SCH₂-, eine Einfachbindung oder eine Alkylengruppe mit 3 bis 6 C-Atomen, worin auch eine CH₂-Gruppe durch -O-, -CO-O-, -O-CO-, -CHHalogen- oder -CHCN- ersetzt sein kann, und
Y O, S, CO, CO-O oder O-CO,
Q (CHF)ₒ-CF₂-(CHF)ₚ, im Falle n=O, auch -CH₂-CF₂. oder -CF₂- CF₂,
m 0, 1 oder 2,
n 0 oder 1, und
0, p 0 oder 1
bedeuten,
mit der Maßgabe, daß falls R² H bedeutet, die Summe von o + p 1 oder 2 ist.

2. Partiell fluorierte Verbindungen der Formel I' 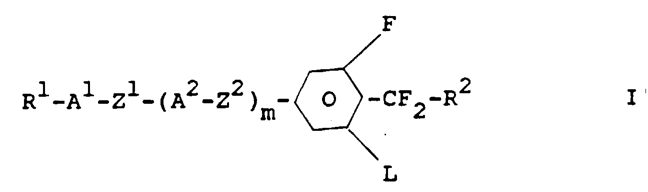 wobei
R¹, R², A¹, A², Z¹, Z² und m die für Formel I angegebene Bedeutung besitzen, und
L H oder F bedeutet.

3. Partiell fluorierte Verbindungen der Formel I" 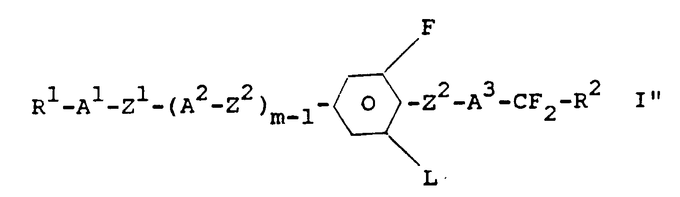 wobei
R¹, R², A¹, A², A³, Z¹, Z² und m die für Formel I angegebene Bedeutung besitzen, und
L H oder F bedeutet.

4. Partiell fluorierte Verbindungen nach Anspruch 1, worin
R¹ und R² jeweils unabhängig voneinander einen Alkylrest mit 1 bis 15 C-Atomen, und
Q CF₂
bedeuten.

5. Partiell fluorierte Verbindungen nach Anspruch 1, worin
R¹, A¹, A², Z¹, Z², und m die angegebene Bedeutung besitzen,
n O,
R² Alkoxy, Thioalkyl, Alkanoyl, Alkanoyloxy oder Alkoxycarbonyl mit 1 bis 14 C-Atomen, und
A³ unsubstituiertes oder durch ein oder zwei Fluor substituiertes 1,4-Phenylen oder trans-1,4-Cyclohexylen
bedeuten.

6. Partiell fluorierte Verbindungen nach Anspruch 4, worin
(Y)ₙ-Q- CO-CF₂-
bedeutet.

7. Partiell fluorierte Verbindungen nach mindestens einen der Ansprüche 1 bis 6, worin mindestens einer der Reste A¹, A² und A³ gegebenenfalls durch Fluor substituiertes 1,4-Phenylen, 1,4-Cyclohexylen, Pyrimidin-2,5-diyl oder Pyridin-2,5-diyl bedeutet.

8. Verwendung der partiell fluorierten Verbindungen nach einem der Ansprüche 1 bis 7 als Komponenten flüssigkristalliner Medien für elektrooptische Anzeigeelemente.

9. Flüssigkristallines Medium mit mindestens zwei Komponenten, dadurch gekennzeichnet, daß mindestens eine Komponente eine partiell fluorierten Verbindung nach einem der Ansprüche 1 bis 7 ist.

10. Elektrooptische Anzeige, dadurch gekennzeichnet, daß sie als Dieelektrikum ein flüssigkristallines Medium nach Anspruch 9 enthält.

11. Matrix-Flüssigkristallanzeige nach Anspruch 10, dadurch gekennzeichnet, daß sie als Dielektrikum ein flüssigkristallines Medium nach Anspruch 9 enthält.

## Claims

1. Partially fluorinated compounds of the formula I
R¹-A¹-Z¹-(A²-Z²)ₘ-A³-(Y)ₙ-Q-R²
where
A¹, A² and A³ are each, independently of one another, a
(a) trans-1,4-cyclohexylene radical in which, in addition, one or more nonadjacent CH₂ groups may be replaced by -O- and/or -S-,
(b) 1,4-phenylene radical in which, in addition, one or two CH groups may be replaced by N,
(c) radical from the group comprising 1,3-cyclobutylene, 1,3-bicycle(1,1,1)pentylene, 1,4-cyclohexenylene, 1,4-bicyclo-(2,2,2)octylene, piperidine-1,4-diyl, naphthalene-2,6-diyl, decahydronaphthalene-2,6-diyl and 1,2,3,4-tetrahydronaphthalene-2,6-diyl,
it being possible for the radicals (a) and (b) to be substituted by CN or halogen,
R¹ and R² are each, independently of one another, an alkyl or alkenyl radical having up to 15 carbon atoms which is unsubstituted or monosubstituted by CN, halogen or CF₃, it also being possible for one or more CH₂ groups in these radicals to be replaced, in each case independently of one another, by -S-, -O-, -CO-, -CO-O-, -O-CO- or -O-CO-O- in such a manner that S and/or O atoms are not linked directly to one another, or one of the radicals R¹ and R² is alternatively H,
Z¹ and Z² are each, independently of one another, -CH₂CH₂-, -C≡C-, -CH₂O-, -OCH₂-, -CO-O-, -O-CO-, -CH=N-, -N=CH-, -CH₂S-, -SCH₂-, a single bond or an alkylene group having 3 to 6 carbon atoms in which, in addition, one CH₂ group may be replaced by -O-, -CO-O-, -O-CO-, -CHHalogen- or -CHCN-,
y is O, S, CO, CO-O or O-CO,
Q is (CHF)ₒ-CF₂-(CHF)ₚ, and, in the case where n = 0, is alternatively -CH₂-CF₂ or -CF₂-CF₂,
m is 0, 1 or 2,
n is 0 or 1, and
o and p are 0 or 1,
with the proviso that, if R² is H, the sum o + p is 1 or 2.

2. Partially fluorinated compounds of the formula I' 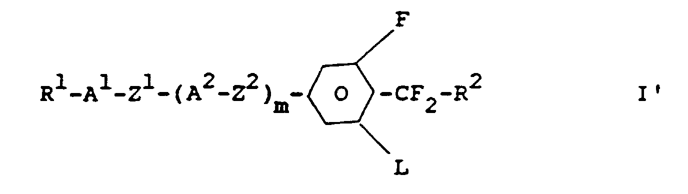 where
R¹, R², A¹, A², Z¹, Z² and m are as defined for the formula I, and
L is H or F.

3. Partially fluorinated compounds of the formula I" 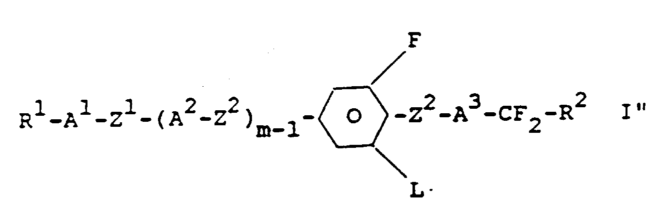 where
R¹, R², A¹, A², A³, Z¹, Z² and m are as defined for the formula I, and
L is H or F.

4. Partially fluorinated compounds according to claim 1,
in which
R¹ and R² are each, independently of one another, an alkyl radical having 1 to 15 carbon atoms, and
Q is CF₂.

5. Partially fluorinated compounds according to claim 1,
in which
R¹, A¹, A², Z¹, Z² and m are as defined above,
n is 0,
R² is alkoxy, thioalkyl, alkanoyl, alkanoyloxy or alkoxycarbonyl having 1 to 14 carbon atoms, and
A³ is unsubstituted or mono- or di-fluorinesubstituted 1,4-phenylene or trams-1,4-cyclohexylene.

6. Partially fluorinated compounds according to claim 4,
in which
(Y)ₙ-Q- is CO-CF₂-.

7. Partially fluorinated compounds according to at least one of claims 1 to 6, in which at least one of the radicals A¹, A² and A³ is optionally fluorinesubstituted 1,4-phenylene, 1,4-cyclohexylene, pyrimidine-2,5-diyl or pyridine-2,5-diyl.

8. Use of the partially fluorinated compounds according to one of claims 1 to 7 as components of liquid-crystalline media for electrooptical display elements.

9. Liquid-crystalline medium containing at least two components, characterized in that at least one component is a partially fluorinated compound according to one of claims 1 to 7.

10. Electrooptical display, characterized in that it contains, as dielectric, a liquid-crystalline medium according to claim 9.

11. Matrix liquid-crystal display according to claim 10, characterized in that it contains, as dielectric, a liquid-crystalline medium according to claim 9.

## Revendications

1. Composés partiellement fluorés de formule I,
R¹-A¹-Z¹-(A²-Z²)ₘ-A³-(Y)ₙ-Q-R²
où
A¹, A¹ et A³ représentent respectivement indépendamment l'un de l'autre
(a) un reste trams-1,4-cydohexyléne, où aussi un ou plusieurs groupes CH₂ non contigus peuvent être remplacés par -O- et/ou -S-,
(b) un reste 1,4-phénylène, où aussi un ou deux groupes CH peuvent être remplacés par N,
(c) un reste du groupe de 1,3-cyclo-butylène, 1,3-bicyclo(1,1,1)-pentylène, 1,4-cyclohexènylène, 1,4-bicyclo(2,2,2)octylène, pipéridine-1,4-diyle, naphtalène-2,6-diyle, déca-hydronaphtalène-2,6-diyle, et 1,2,3,4-tétrahydronaphtalène-2,6-diyle,
où les restes (a) et (b) peuvent être substitués par CN ou halogène,
R¹ et R² respectivement indépendamment l'un de l'autre représentent un reste alkyle ou alkylène pouvant avoir jusqu'à 15 atomes de C, non substitué ou substitué une fois par CN, halogène ou CF₃, où dans ces restes aussi un ou plusieurs groupes CH₂ peuvent être remplacés respectivement indépendamment l'un de l'autre par -S-, -O-, -CO-,-CO- O-, -O-CO- ou -O-CO-O-, de sorte que les atomes S et/ou O ne sont pas reliés directement l'un à l'autre, l'un des restes R₁ et R₂ peut être aussi H,
Z¹ et Z² représentent respectivement indépendamment l'un de l'autre -CH₂CH₂-, -C≡C-, -CH₂O-, -OCH₂-, -CO-O-, -O-CO-, -CH=N-, -N=CH-,-CH₂S-, -SCH₂-, une liaison simple ou un groupe alkylène de 3 à 6 atomes de C,
où aussi un groupe CH₂ peut être remplacé par -O-, -CO-O-, -O-CO-,-CHHalogène- ou -CHCN-, et
Y est 0, S, CO, CO-O ou O-CO,
Q est (CHF)ₒ-CF₂- (CHF)ₚ, si n = 0, aussi -CH₂CF₂ Ou -CF₂-CF₂, m est 0, 1 ou 2,
n est 0 ou 1, et
o, p représentent 0 ou 1
avec la condition,
que si R² représente Ht la somme de o + p est 1 ou 2.

2. Composés partiellement fluorés de formule l' 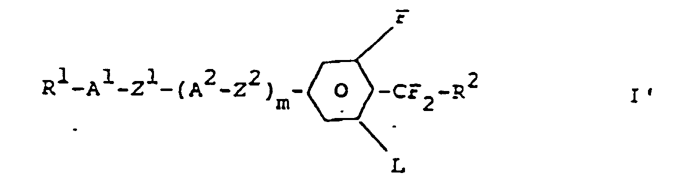 où
R¹, R², A¹, A², Z¹, Z² et m ont les significations données pour la formule I, et L représente H ou F.

3. Composés partiellement fluorés de formule I" 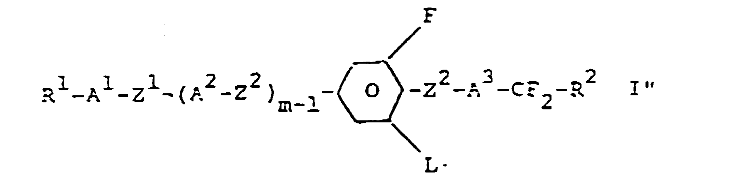 où
R¹, R², A¹, A¹, A³, Z¹, Z² et m ont les significations données pour la formule I et
L représente H ou F.

4. Composés partiellement fluorés selon la revendication 1 où
R¹ et R² représentent respectivement indépendamment l'un de l'autre un reste alkyle avec à 15 atomes de C,
Q est CF₂.

5. Composés partiellement fluorés selon la revendication 1, où R¹, A¹, A², Z¹, Z² et m ont les significations données,
n est 0,
R² est un alcoxy, thioalkyle, alcanoyle, alcanoyloxy ou alcoxycarbonyle de 1 à 14 atomes de C, et
A³ est un 1,4-phénylène ou trans-1,4-cyclo-hexylène non substitué ou substitué par 1 ou 2 fluor.

6. Composés partiellement fluorés selon la revendication 4, où
(Y)ₙ-Q)- représente CO-CF₂-

7. Composés partiellement fluorés selon au moins l'une des revendications 1 à 6, où au moins l'un des restes A¹, A² et A³ représente un 1,4-phénylène, 1,4-cyclohexylène, pyrimidine-2,5-diyle, ou pyridine-2,5-diyle substitué le cas échéant par du fluor.

8. Utilisation des composés partiellement fluorés selon l'une des revendications 1 à 7, comme composants de milieux de cristaux liquides pour des éléments d'afficheurs électrooptiques.

9. Milieu de cristaux liquides avec au moins deux composants dont au moins un composant est un composé partiellement fluoré selon l'une des revendications 1 à 7.

10. Afficheur électrooptique, caractérisé en ce qu'il contient comme diélectrique un milieu de cristaux liquides selon la revendication 9.

11. Afficheur à cristaux liquides à matrice selon la revendication 10, caractérisé en ce qu'il contient comme diélectrique un milieu de cristaux liquides selon la revendication 9.
